# EUROPÄISCHE PATENTSCHRIFT

(11) **EP 3 188 233 B1**
(45) Veröffentlichungstag und Bekanntmachung des Hinweises auf die Patenterteilung: **02.10.2019**
(21) Anmeldenummer: 16002582.1
(22) Anmeldetag: 05.12.2016
(51) Int. Cl.: H01L 27/02, A61B 5/053

(54) **AKTIVE SCHUTZSCHALTUNG FÜR EINEN MESSVERSTÄRKER IN EINEM ELEKTRODENGÜRTEL FÜR EINEN ELEKTRISCHEN IMPEDANZTOMOGRAPHEN**
ACTIVE CIRCUIT PROTECTION FOR A MEASUREMENT REINFORCER IN AN ELECTRODE BELT FOR AN ELECTRICAL IMPEDANCE TOMOGRAPH
CIRCUIT DE PROTECTION ACTIF POUR UN AMPLIFICATEUR DE MESURE DANS UNE BANDE D'ÉLECTRODES POUR UN TOMOGRAPHE À IMPÉDANCE ÉLECTRIQUE

(30) Priorität: 11.12.2015 DE 102015016091
(43) Veröffentlichungstag der Anmeldung: 05.07.2017
(73) Patentinhaber: Drägerwerk AG & Co. KGaA, 23558 Lübeck (DE)
(72) Erfinder: Li, Jianhua, 23558 Lübeck (DE); Sattler, Frank, 23558 Lübeck (DE); Hiltawsky, Karsten, 23617 Stockelsdorf (DE)

(56) Entgegenhaltungen:
- EP-A1- 0 848 472
- WO-A1-98/49444
- WO-A1-2009/146516
- ES-T3- 2 366 378
- JP-A- H06 237 124
- US-A- 5 196 980
- US-A- 5 365 099
- US-A- 5 536 958
- US-A- 5 642 252
- US-A1- 2007 049 993
- US-A1- 2008 258 224
- US-A1- 2014 100 637
- US-A1- 2014 240 036
- US-A1- 2015 263 504
- US-A1- 2015 325 568
- CHANG-MING YANG ET AL: "Performance assessment of active electrode applied in wearable physiological monitoring system", BIOMEDICAL AND HEALTH INFORMATICS (BHI), 2012 IEEE-EMBS INTERNATIONAL CONFERENCE ON, IEEE, 5. Januar 2012 (2012-01-05), Seiten 472-474, XP032449106, DOI: 10.1109/BHI.2012.6211619 ISBN: 978-1-4577-2176-2

## Beschreibung

Die vorliegende Erfindung betrifft einen Elektrodengürtel für einen elektrischen Impedanztomographen mit einer Mehrzahl von Elektroden, einer Mehrzahl von Messverstärkern und einer Mehrzahl von aktiven Schutzschaltungen für jeden der Mehrzahl von Messverstärkern.

Bei der elektrischen Impedanztomographie wird die elektrische Leitfähigkeit eines menschlichen oder tierischen Körpers zwischen einer Vielzahl von Elektroden bestimmt und hieraus eine optische Darstellung des Körpers abgeleitet. Zur Messung der elektrischen Leitfähigkeit wird ein so genannter Elektrodengürtel mit einer Mehrzahl von Elektroden auf der Hautoberfläche des Körpers angeordnet. Zwischen zwei Elektroden wird jeweils ein Wechselstrom, beispielsweise mit einer Frequenz zwischen 10 und 200 kHz, mit einer Amplitude von wenigen Milliampere angelegt. Die Ausbreitung dieses Wechselstroms wird von den übrigen Elektroden in Form des Spannungsabfalls zwischen den Elektroden erfasst und als Grundlage für die Berechnung eines Schnittbildes in einem Impedanztomographen oder elektrischen Impedanztomographen verwendet. Die genaue Messung und Verstärkung der gemessenen Spannungen setzt eine hochempfindliche Elektronik voraus.

Die elektrische Impedanztomographie wird heutzutage unter anderem dazu verwendet, die Lungenfunktion von beatmeten Patienten, beispielsweise Patienten auf einer Intensivstation, zu überwachen. Kommt es bei solchen Patienten zu einem Herzstillstand, wird ein Defibrillator verwendet, um das Herz durch gezielte Stromstöße wieder zu aktivieren. Dabei werden kurzfristig Spannungen von bis zu 5 kV an den Patienten angelegt. Für derartig hohe Spannungen ist die hochempfindliche Elektronik des elektrischen Impedanztomographen nicht ausgelegt. Da es allerdings in der Regel auch an der Zeit fehlen wird, sämtliche Elektroden vom Patienten zu entfernen, bevor der Defibrillator eingesetzt wird, müssen andere Schutzmaßnahmen verwendet werden, um die Elektronik des Impedanztomographen vor dem Stromstoß des Defibrillators zu schützen. Darüber hinaus ist es auch notwendig, den Impedanztomographen automatisch von dem Patienten zu trennen, um zu verhindern, dass der Impedanztomographen dem Stromstoß des Defibrillators zu viel Energie entzieht und der Stromstoß eventuell nicht mehr ausreichend stark ist, um das flimmernde oder flatternde Herz still zu setzen.

Aus der US 2014/0240036 A1 ist eine Halbleitervorrichtung mit einem N-Kanal-MOS-Transistor und einer Steuerspannungserzeugungsschaltung zur Verwendung bei Kraftfahrzeugen bekannt. Der N-Kanal-MOS-Transistor steuert die Zufuhr einer Versorgungsspannung auf Basis einer von der Steuerspannungserzeugungsschaltung erzeugten Steuerspannung.

Die US 2015/0263504 A1 beschreibt eine Puffer- oder Spannungsschutzschaltung. In einer beispielhaften Ausführungsform umfasst eine integrierte Schaltung einen ersten Eingangsanschluss, einen ersten Schaltungsabschnitt mit einem zweiten Eingangsanschluss und einen zweiten Schaltungsabschnitt. Der zweite Schaltungsabschnitt enthält eine Transistorvorrichtung mit einem ersten, einem zweiten und einem dritten Anschluss, wobei der erste und der zweite Anschluss jeweils mit dem ersten Eingangsanschluss bzw. dem zweiten Eingangsanschluss elektrisch verbunden sind. Zusätzlich umfasst der zweite Schaltungsabschnitt auch eine Diode, die elektrisch zwischen dem dritten Anschluss und entweder einer Stromquelle oder einem Leistungseingangsanschluss gekoppelt ist, und eine Puffer/Treiber-Schaltung und einen Kondensator, die in Reihe zwischen dem dritten und dem zweiten Anschluss geschaltet sind. Der zweite Schaltungsabschnitt dient dazu, zu verhindern, dass der zweite Eingangsanschluss einem unerwünscht hohen Spannungspegel ausgesetzt wird.

Die US 5,196,980 A offenbart eine Niedrigimpedanz-Überspannungsschutzschaltung, die einen ersten MOSFET mit einem Drain enthält, der mit einem Eingangssignal verbunden ist, und einer Source, die mit einem Drain eines zweiten MOSFET verbunden ist, wobei die Source des zweiten MOSFET mit dem Ausgang verbunden ist. Die Gates der ersten und zweiten MOSFETs sind mit Spannungsversorgungen verbunden, die relativ zu den Eingangssignalwerten schweben, um so die Gates der jeweiligen MOSFETs in einem leitenden Zustand zu halten. Wenn der Eingangssignalwert einen gewünschten positiven Maximalwert überschreitet, wird der erste MOSFET nicht länger in einem Ein-Zustand gehalten, wodurch der MOSFET ausschaltet, wodurch das Eingangssignal vom Ausgang entfernt wird. Negative Spitzenwerte werden in ähnlicher Weise durch den zweiten MOSFET entfernt.

Die US 2015/0325568 A1 betrifft eine Schutzschaltung gegen elektrostatische Entladungen (ESD). Offenbart ist eine ESD-Schutzschaltung, um eine integrierte Schaltung (IC) während der Herstellung und Produktion zu schützen. Eine ESD-Detektionsschaltung erfasst ein ESD-Ereignis durch Detektieren einer Spannungsspitze zwischen einer Versorgungsschiene und einer Erdungsschiene, die eine ESD-Schwellenspannung überschreitet. In Reaktion auf das Erfassen des ESD-Ereignisses wird eine ESD-Klemmschaltung aktiviert, um das ESD-Ereignis zu entladen, wodurch die integrierte Schaltung vor einer Beschädigung durch das ESD-Ereignis geschützt wird.

Gemäß der DE 20 2005 013 792 U1 ist bekannt, eine Schutzschaltung für ein EIT-Gerät vorzusehen, die eine Sicherung zwischen der Elektrode am Patienten und dem Signaleingang am EIT-Gerät aufweist. In die Sicherung ist eine Reihenschaltung aus zwei Widerständen eingefügt, die sich vor der ersten Verstärkerstufe des EIT-Geräts befindet. Unmittelbar nach dem Beginn einer Defibrillation werden die Elektroden mittels der Sicherung schnell von dem EIT-Gerät getrennt. Nachteilig ist, dass die Sicherung nur ab einer gewissen Energiehöhe des Defibrillator-Pulses funktioniert und diese Sicherung auf Grund ihrer Größe lediglich am EIT-Gerät und nicht am Elektrodengürtel implementiert werden kann.

Aus der DE 10 2008 002 330 A1 ist ein Überspannungsschutzelement bekannt, das als Überspannungsschutzhebel für ein medizinisches Gerät am Körper eines Patienten ausgebildet sein kann. Dabei wird das Überspannungsschutzelement an ein medizinisches Gerät mit einer bipolaren Schnittstelle mit einem ersten und einem zweiten Eingang angeschlossen. Das Überspannungsschutzelement umfasst nachgeschaltet an den Eingängen einen ersten und einen zweiten Kondensator als Umformungsmittel. Nachgeschaltet sind zwei Zener-Dioden, die in entgegengesetzter Polung in Reihe geschaltet sind und beide Eingangsleitungen elektrisch verbinden. Das Überspannungsschutzelement ist lediglich an der Schnittstelle am medizinischen Gerät vorgesehen und nicht direkt an einem Elektrodengürtel.

Aus der DE 10 2005 041 385 A1 ist eine Schutzschaltung für einen elektrischen Impedanztomographen bekannt. Diese Schutzschaltung aus einem in Reihe geschalteten RC-Glied mit einem Kondensator und einem Widerstand sichert zusammen mit einer Funkenstrecke die Signaleingänge des Impedanztomographen im normalen Messbetrieb gegen zu hohe Spannungen ab. Dabei dient der Kondensator als eigentlicher Schutz vor dem eigentlichen Stromstoß. Damit die Funktionsfähigkeit des Impedanztomographen nicht gefährdet wird, muss der Kondensator des RC-Glieds mindestens eine Spannungsfestigkeit von 5 kV aufweisen. Um die Spannungsfestigkeit zu gewährleisten, muss der Kondensator eine minimale Baugröße aufweisen, die den Kondensator zu dem Bauelement der Schutzschaltung mit dem größten Bauvolumen macht. Die Mindestgröße des Kondensators führt jedoch dazu, dass es aus Platzgründen mehr oder weniger unmöglich ist, die Schutzschaltung direkt in den Elektrodengürtel zu implementieren. Da tatsächlich jede der Elektroden des Impedanztomographen doppelt abgesichert werden muss, wird das gesamte Bauvolumen für die Schutzschaltung weiter erhöht.

Die WO 2009/146516 beschreibt ein EIT-System mit Schutz gegen Hochspannungswerte, die durch die Verwendung eines externen Defibrillators entstehen.

Die US 2014/0100637 beschreibt ein implantierbares medizinisches Gerät mit einer Hochspannungsschutz-Steuerschaltung in jedem Eingangskanal. Die Schutzschaltung aktiviert den Hochspannungsschutz, wenn sie einen Hinweis darauf erhält, dass das implantierbare medizinische Gerät eine Defibrillations-Schocktherapie erzeugt.

Da es mehr oder weniger unmöglich ist, die Schaltung direkt in den Elektrodengürtel zu implementieren, wird diese heutzutage in der Regel direkt im Impedanztomographen angeordnet. Damit müssen jedoch sämtliche Komponenten, von der Elektrode bis hin zu den Leitungen, die zum Impedanztomographen führen, eine Spannungsfestigkeit von wenigstens 5 kV aufweisen. Zudem müssen die Komponenten sämtlich so ausgebildet sein, dass für medizinisches Personal, das sich in der Nähe des Impedanztomographen aufhält, keine Gefahr durch den Defibrillatorpuls entsteht. Daher sind die Komponenten des Elektrodengürtels verhältnismäßig aufwendig in der Herstellung und teuer. Zudem wäre es aufgrund der niedrigen Spannungen, die von den Elektroden erfasst werden müssen, vorteilhaft, aktive Verstärkerkomponenten direkt in dem Elektrodengürtel anzuordnen. Bei den heutigen Lösungen müssten diese aktiven Verstärkerkomponenten ebenfalls eine Spannungsfestigkeit von wenigstens 5 kV aufweisen, damit sie nicht durch den Defibrillatorpuls zerstört werden. Da die aktiven Verstärkerkomponenten sonst dazu ausgelegt sind, Spannungen mit einer Amplitude von wenigen mV oder µV zu verstärken, erscheint es sehr schwierig, gleichzeitig eine Spannungsfestigkeit für einen Defibrillatorpuls zu gewährleisten.

Es ist daher unter anderem die Aufgabe der vorliegenden Erfindung, eine aktive Schutzschaltung zur Verwendung mit einem Elektrodengürtel für einen Impedanztomographen bereitzustellen, deren Baugröße deutlich geringer ist als die Baugröße bereits bekannter Schutzschaltungen. Vorzugsweise soll die Schutzschaltung dazu geeignet sein, direkt in einem Elektrodengürtel für einen Impedanztomographen eingesetzt zu werden und aktive Verstärkerkomponenten in einem solchen Elektrodengürtel zu schützen.

Gemäß einem Aspekt wird die der Erfindung zugrunde liegende Aufgabe durch einen Elektrodengürtel für einen elektrischen Impedanztomographen mit den Merkmalen von Anspruch 1 gelöst. Dieser Elektrodengürtel weist eine Mehrzahl von Elektroden, eine Mehrzahl von Messverstärkern und eine Mehrzahl von aktiven Schutzschaltungen für jeden der Mehrzahl von Messverstärkern auf. Jeder der Mehrzahl von Elektroden ist zumindest eine der Mehrzahl von aktiven Schutzschaltungen zugeordnet, und jede der Mehrzahl von aktiven Schutzschaltungen ist nur einer der Mehrzahl von Elektroden zugeordnet. Jede der Mehrzahl von Elektroden ist über zumindest eine der Mehrzahl von aktiven Schutzschaltungen mit einem der Mehrzahl von Messverstärkern verbunden. Jede der Mehrzahl von aktiven Schutzschaltungen weist eine Schaltelementanordnung auf, die einen Elektrodeneingang und einen Ausgang sowie zumindest einen Steuereingang für eine Steuerspannung aufweist. Der Elektrodeneingang der Schaltelementanordnung ist mit einer der Mehrzahl von Elektroden verbunden, und der Ausgang der Schaltelementanordnung ist mit einem der Mehrzahl von Messverstärkern verbunden.

Die Schaltelementanordnung ist derart ausgebildet, dass die Schaltelementanordnung eine leitende Verbindung zwischen dem Elektrodeneingang der Schaltelementanordnung und dem Ausgang der Schaltelementanordnung ausbildet, wenn eine an dem zumindest einen Steuereingang anliegende Steuerspannung in einem ersten Spannungsbereich ist. Die Schaltelementanordnung ist weiterhin derart ausgebildet, dass die Schaltelementanordnung keine leitende Verbindung zwischen dem Elektrodeneingang der Schaltelementanordnung und dem Ausgang der Schaltelementanordnung ausbildet, wenn die an dem zumindest einen Steuereingang anliegende Steuerspannung in einem zweiten Spannungsbereich ist. Die Schutzschaltung ist so ausgebildet, dass die an dem zumindest einen Steuereingang anliegende Spannung in dem zweiten Spannungsbereich ist, wenn am Elektrodeneingang eine Spannung anliegt, die in einem Abschaltbereich liegt.

Mit anderen Worten umfasst die erfindungsgemäße aktive Schutzschaltung eine Schaltelementanordnung, die zumindest drei Eingänge aufweist: einen Elektrodeneingang, einen Ausgang und zumindest einen Steuereingang für eine Steuerspannung. Diese Schaltelementanordnung kann beispielsweise direkt in einem Elektrodengürtel für einen Impedanztomographen angeordnet sein. Es ist allerdings auch denkbar, dass die Schutzschaltung beispielsweise in dem eigentlichen Impedanztomographen angeordnet wird. In jedem Fall ist der Elektrodeneingang aber zur Verbindung mit einer Elektrode ausgebildet, d.h., im Betrieb wird der Elektrodeneingang elektrisch mit einer Elektrode verbunden. Entsprechend ist der Ausgang der Schaltelementanordnung zur Verbindung mit einem Messverstärker ausgebildet, d.h., im Betrieb wird der Ausgang elektrisch mit dem Messverstärker verbunden. Bei dem Messverstärker kann es sich beispielsweise um einen lokalen Vorverstärker handeln, der direkt in einem Elektrodengürtel angeordnet ist. Alternativ ist aber auch denkbar, dass der Messverstärker in dem eigentlichen Impedanztomographen angeordnet ist, auch wenn die aktive Schutzschaltung selbst in dem Elektrodengürtel angeordnet ist.

Die Schaltelementanordnung ist erfindungsgemäß dazu ausgebildet, in zwei verschiedenen Zuständen zu arbeiten. Daher kann die Schaltelementanordnung beispielsweise elektronische Bauelemente aufweisen, die nur in zwei Zuständen betrieben werden können, wie beispielsweise MOSFET (Metall-Oxid-Halbleiter-Feldeffekttransistor). Diese Schaltelementanordnung ist so ausgebildet, dass sie den Elektrodeneingang mit dem Ausgang dann leitend verbindet, wenn die Steuerspannung, d.h., die an dem Steuereingang anliegende Spannung, in einem ersten Spannungsbereich ist. Liegt die Steuerspannung in einem zweiten, nicht mit dem ersten Spannungsbereich überlappenden Spannungsbereich, dann bildet die Schaltelementanordnung keine leitende Verbindung zwischen dem Elektrodeneingang der Schaltelementanordnung und dem Ausgang der Schaltelementanordnung aus. Mit anderen Worten leitet die Schaltelementanordnung ein an dem Elektrodeneingang anliegendes Signal nicht an den Ausgang und damit an den nachgeordneten Messverstärker weiter.

Ob die Steuerspannung im ersten oder im zweiten Spannungsbereich liegt, hängt davon ab, wie hoch die Spannung ist, die am Elektrodeneingang anliegt. Beispielsweise kann die Schutzschaltung so ausgebildet sein, dass die Steuerspannung dauerhaft im ersten Spannungsbereich liegt, wenn die an dem Elektrodeneingang anliegende Spannung in einem Bereich liegt, der im normalen Messbetrieb des Impedanztomographen verwendet wird. Die Schaltelementanordnung verbindet dann den Elektrodeneingang leitend mit dem Ausgang und leitet die gemessenen Spannungen direkt an den Messverstärker weiter. Liegt jedoch an dem Elektrodeneingang eine Spannung an, die in einem vorbestimmten Abschaltbereich ist, so wechselt die Steuerspannung automatisch in den zweiten Bereich und die Verbindung zwischen dem Elektrodeneingang und dem Ausgang der Schaltelementanordnung wird getrennt. Damit werden Spannungen, die in Verbindung mit Spannungspulsen stehen, die im Abschaltbereich liegen, nicht von der Schaltelementanordnung weitergeleitet und belasten damit auch keine nachgeordneten Messverstärker. Der Abschaltbereich kann beispielsweise durch eine untere Spannungsgrenze, beispielsweise 20 V, definiert sein und sich von dieser unteren Spannungsgrenze als gegen unendlich offener Bereich darstellen. Es ist allerdings auch denkbar, dass der Abschaltbereich zwei Abschnitte aufweist, beispielsweise einen ersten Abschnitt, der sämtliche Spannungen kleiner -20 V umfasst, und einem zweiten Abschnitt, der sämtliche Spannungen größer 20 V umfasst.

Die Schaltelementanordnung der aktiven Schutzschaltung ist damit auf vorteilhafte Weise so ausgestaltet, dass sie die Verbindung zwischen dem Elektrodeneingang und dem Ausgang trennt, sobald die an dem Elektrodeneingang anliegende Spannung in einen Abschaltbereich wechselt, in dem der nachgeordnete Messverstärker nicht mehr sicher betrieben werden kann. Wird also beispielsweise ein Defibrillator zur Wiederbelebung eines Patienten verwendet, an dem ein Elektrodengürtel für einen Impedanztomographen mit einer erfindungsgemäßen aktiven Schutzschaltung angelegt ist, so trennt die aktive Schutzschaltung, genauer gesagt, die Schaltelementanordnung, die Verbindung zwischen dem Elektrodeneingang und dem Ausgang, sobald die am Elektrodeneingang anliegende Spannung in den Abschaltbereich wechselt. Wird der Abschaltbereich hinreichend breit definiert, so trennt die Schaltelementanordnung den Elektrodeneingang bereits am unteren Ende der ansteigenden Flanke des Defibrillatorpulses ab und verhindert so eine Beschädigung der nachgeschalteten Elektronik. Da die Schaltelementanordnung keinen Kondensator aufweist, der den eigentlichen Defibrillatorpuls aufnehmen muss, kann diese deutlich kleiner ausfallen, als aus dem Stand der Technik bekannte Schutzschaltungen.

In einer bevorzugten Ausführungsform ist der Ausgang der Schaltelementanordnung mit dem zumindest einem Steuereingang der Schaltelementanordnung über ein Sperrelement verbunden. Das Sperrelement ist so ausgebildet, dass die an dem Ausgang der Schaltelementanordnung anliegende Spannung nur dann an den Steuereingang angelegt wird, wenn die am Ausgang der Schaltelementanordnung anliegende Spannung im Abschaltbereich ist.

Mit anderen Worten besteht zwischen dem Ausgang der Schaltelementanordnung und dem Steuereingang der Schaltelementanordnung eine Verbindung. Diese Verbindung umfasst zumindest ein elektronisches Bauteil, das abhängig von der am Ausgang der Schaltelementanordnung anliegenden Spannung entweder leitend oder nicht-leitend ist. Beispielsweise kann es sich bei dem Bauteil um eine Zenerdiode oder Z-Diode handeln, die so zwischen dem Ausgang und dem Steuereingang angeordnet ist, dass sie im normalen Messbetrieb sperrt und dann leitend wird, wenn die am Ausgang - und damit an der Z-Diode - anliegende Spannung in den Abschaltbereich wechselt. Sobald das Sperrelement leitend wird, wird die am Ausgang der Schaltelementanordnung anliegende Spannung auch an den Steuerausgang gelegt. Auf bevorzugte Weise werden das Sperrelement und eventuelle weitere Komponenten in der Verbindung zwischen dem Ausgang der Schaltelementanordnung und dem Steuereingang so gewählt, dass die am Steuereingang anliegende Spannung genau dann in den zweiten Bereich wechselt, wenn das Sperrelement leitend ist und die am Ausgang der Schaltelementanordnung anliegende Spannung in den Abschaltbereich wechselt. Dies führt auf vorteilhafte Weise dazu, dass die am Ausgang anliegende Spannung direkt als Schaltschwelle verwendet wird, um die Schaltelementanordnung so zu schalten, dass der Elektrodeneingang nicht mehr leitend mit dem Ausgang verbunden ist.

Es ist weiterhin bevorzugt, wenn die aktive Schutzschaltung parallel zu der Schaltelementanordnung eine Bypassschaltung aufweist, die den Eingang der Schaltelementanordnung mit dem Ausgang der Schaltelementanordnung verbindet. Die Bypassschaltung ist so ausgebildet, dass an dem Ausgang der Schaltelementanordnung eine Spannung im Abschaltbereich anliegt, wenn die Schaltelementanordnung keine leitende Verbindung zwischen dem Elektrodeneingang der Schaltelementanordnung und dem Ausgang der Schaltelementanordnung ausbildet, und die an dem Eingang der Schaltelementanordnung anliegende Spannung in einem Hochvoltbereich ist, wobei der Hochvoltbereich ein Teilbereich des Abschaltbereichs ist. Auf bevorzugte Weise kann die Bypassschaltung beispielsweise durch einen hochohmigen Widerstand gebildet werden. Die optionale Bypassschaltung stellt auf vorteilhafte Weise sicher, dass auch wenn die Schaltelementanordnung den Elektrodeneingang von dem Ausgang der Schaltelementanordnung getrennt hat, an der Verbindung zwischen dem Ausgang der Schaltelementanordnung und dem Steuereingang weiterhin eine Spannung anliegt, die so hoch ist, dass die Steuerspannung im zweiten Bereich gehalten wird und die Verbindung zwischen dem Elektrodeneingang und dem Ausgang der Schaltelementanordnung dauerhaft getrennt bleibt. Es hat sich allerdings herausgestellt, dass die Bypassschaltung nicht zwingend notwendig ist, um die erfindungsgemäße aktive Schutzschaltung sicher zu betreiben.

In einer bevorzugten Ausführungsform umfasst die aktive Schutzschaltung eine Speicherschaltung, die so ausgebildet ist, dass an dem zumindest einen Steuereingang dauerhaft eine Spannung angelegt wird, die im zweiten Spannungsbereich ist, wenn an dem Elektrodeneingang einmal eine Spannung anliegt, die im Abschaltbereich ist. Mit anderen Worten ist die Speicherschaltung so ausgebildet, dass beim einmaligen Überschreiten der Grenzen des Abschaltbereichs die am Steuereingang angelegte Spannung dauerhaft in dem Spannungsbereich gehalten wird, in dem die Schaltelementanordnung den Elektrodeneingang von dem Ausgang trennt.

Es ist weiterhin bevorzugt, wenn die Schaltelementanordnung ein erstes Schaltelement mit einem Eingang, einem Ausgang und einem Steuereingang aufweist. Der Eingang des ersten Schaltelements ist mit dem Elektrodeneingang verbunden. Der Ausgang des ersten Schaltelements ist mit dem Ausgang der Schaltelementanordnung verbunden. Der Steuereingang des ersten Schaltelements ist mit dem zumindest einem Steuereingang der Schaltelementanordnung verbunden. Das erste Schaltelement ist so ausgebildet, dass das Schaltelement eine leitende Verbindung zwischen dem Eingang des ersten Schaltelements und dem Ausgang des ersten Schaltelements ausbildet, wenn eine an dem Steuereingang des ersten Schaltelements anliegende Steuerspannung in dem ersten Spannungsbereich ist, und das erste Schaltelement keine leitende Verbindung zwischen dem Eingang des ersten Schaltelements und dem Ausgang des ersten Schaltelements ausbildet, wenn die an dem Steuereingang des ersten Schaltelements anliegende Steuerspannung im zweiten Spannungsbereich ist. Die Schutzschaltung ist so ausgebildet, dass die an dem zumindest einen Steuereingang anliegende Spannung in den zweiten Spannungsbereich wechselt, wenn an dem Elektrodeneingang eine Spannung anliegt, die einen vorbestimmten ersten Schwellwert überschreitet.

Mit anderen Worten umfasst die Schaltelementanordnung zumindest ein erstes Schaltelement, das einen Eingang, einen Ausgang und einen Steuereingang aufweist. Der Eingang des ersten Schaltelements ist mit dem Elektrodeneingang der Schaltelementanordnung verbunden, wobei zwischen dem Eingang des ersten Schaltelements und dem Elektrodeneingang der Schaltelementanordnung weitere Elemente angeordnet sein können. Der Ausgang des ersten Schaltelements ist mit dem Ausgang der Schaltelementanordnung verbunden, wobei auch zwischen dem Ausgang des ersten Schaltelements und dem Ausgang der Schaltelementanordnung weitere Elemente angeordnet sein können. Schließlich ist der Steuereingang des ersten Schaltelements mit dem zumindest einem Steuereingang der Schaltelementanordnung verbunden. Das erste Schaltelement ist so ausgebildet, dass es den Eingang des ersten Schaltelements und den Ausgang des ersten Schaltelements voneinander trennt, wenn die am Steuereingang des ersten Schaltelements anliegende Steuerspannung im zweiten Spannungsbereich ist, und miteinander elektrisch verbindet, wenn die am Steuereingang des ersten Schaltelements anliegende Steuerspannung im ersten Spannungsbereich ist. Dabei ist die Schaltelementanordnung und damit auch das erste Schaltelement so ausgebildet, dass der Wechsel vom ersten in den zweiten Spannungsbereich beim ersten Schaltelement genau dann auftritt, wenn die am Elektrodeneingang anliegende Spannung einen vorbestimmten ersten Schwellwert überschreitet. Mit anderen Worten ist das erste Schaltelement dazu ausgebildet, die Verbindung zwischen dem Elektrodeneingang der Schaltelementanordnung und dem Ausgang der Schaltelementanordnung genau dann zu trennen, wenn die am Elektrodeneingang anliegende Spannung einen positiven Schwellwert überschreitet. Dieser positive Schwellwert kann beispielsweise mit einer unteren Grenze eines nach oben offenen Abschnitts des Abschaltbereichs übereinstimmen.

Das erste Schaltelement ist vorzugsweise ein normal sperrender n-Kanal MOSFET mit einem Drain-Anschluss, einem Source-Anschluss und einem Gate-Anschluss. Der Drain-Anschluss bildet den Eingang des ersten Schaltelements, der Source-Anschluss bildet den Ausgang des ersten Schaltelements und der Gate-Anschluss bildet den Steuereingang des ersten Schaltelements. Das erste Schaltelement ist damit nur dann leitend, wenn an dem Gate-Anschluss des n-Kanal MOSFET eine positive Steuerspannung anliegt. Liegt keine oder eine negative Steuerspannung an dem Gate-Anschluss an, so sperrt der n-Kanal MOSFET. Die Verwendung eines normal sperrenden n-Kanal MOSFET ist bevorzugt, da die aktive Schutzschaltung hierdurch eigensicher wird, d.h., die Schutzschaltung schützt einen nachgeordneten Messverstärker auch dann, wenn sie gerade nicht mit einer Steuerspannung versorgt wird, also selbst nicht aktiv ist.

In einer bevorzugten Ausführungsform ist der Ausgang des ersten Schaltelements mit dem Steuereingang des ersten Schaltelements über das Sperrelement verbunden, wobei das Sperrelement so ausgebildet ist, dass die an dem Ausgang des ersten Schaltelements anliegende Spannung nur dann an dem Steuereingang des ersten Schaltelements angelegt wird, wenn die an dem Ausgang des ersten Schaltelements anliegende Spannung im Abschaltbereich ist. Das Sperrelement wird von zwei in Reihe geschalteten Z-Dioden gebildet, wobei jede Z-Diode eine Durchlass- und eine Sperrrichtung aufweist und wobei die zwei in Reihe geschalteten Z-Dioden so verschaltet sind, dass die Durchlassrichtung der einen Z-Diode der Sperrrichtung der anderen Z-Diode entspricht. Die Verwendung von zwei geeignet gewählten und entgegengesetzt geschalteten Z-Dioden hat zum einen den Vorteil, dass die eine Z-Diode verhindert, dass die dauerhaft am Steuereingang des ersten Schaltelements angeordnete Spannungsversorgung nicht auch am Ausgang des ersten Schaltelements und damit an dem nachgeordneten Messverstärker anliegt. Zum anderen wird aber eben auch sichergestellt, dass die am Steuereingang anliegende Spannung in den zweiten Bereich wechselt, wenn die am Ausgang des ersten Schaltelements anliegende Spannung in den Abschaltbereich wechselt bzw. den vorbestimmten ersten Schwellwert überschreitet.

Es ist weiterhin bevorzugt, wenn die aktive Schutzschaltung parallel zu der Schaltelementanordnung optional die Bypassschaltung aufweist, die den Eingang der Schaltelementanordnung mit dem Ausgang des ersten Schaltelements verbindet, wobei die Schaltelementanordnung so ausgebildet ist, dass an dem Ausgang des ersten Schaltelements eine Spannung im Abschaltbereich anliegt, wenn das erste Schaltelement keine leitende Verbindung zwischen dem Eingang der Schaltelementanordnung dem Ausgang des ersten Schaltelements ausbildet, und die am Eingang der Schaltelementanordnung anliegende Spannung im Hochvoltbereich ist. Die Bypassschaltung weist zumindest einen hochohmigen Widerstand auf. Diese Ausführungsform der Bypassschaltung bringt die bereits beschriebenen Vorteile einer Bypassschaltung mit sich.

Alternativ ist es bevorzugt, wenn die Speicherschaltung so ausgebildet ist, dass an dem Steuereingang des ersten Schaltelements dauerhaft eine Spannung angelegt wird, die im zweiten Spannungsbereich ist, wenn an dem Elektrodeneingang einmal eine Spannung anliegt, die im Abschaltbereich ist. Die Speicherschaltung wird durch ein Flipflop gebildet. Die Speicherschaltung umfasst vorzugsweise ein aktives Bauteil in Form eines Flipflops, das zwei Zustände einnehmen kann und zwischen den beiden Zuständen wechselt, wenn es an einem Triggereingang ein Signal empfängt. Je nach dem, in welchem der zwei Zustände das Flipflop sich befindet, liegt an einem Steuerausgang des Flipflops eine andere Spannung an.

Beispielsweise ist die Speicherschaltung so ausgestaltet, dass in einem ersten Zustand keine von der Speicherschaltung bereitgestellte Spannung an dem Steuereingang des ersten Schaltelements anliegt und in einem zweiten Zustand an dem Steuereingang des ersten Schaltelements eine Spannung anliegt, die dazu führt, dass die einem Steuereingang des ersten Schaltelements anliegende resultierende Spannung im zweiten Spannungsbereich ist. Das erste Schaltelement trennt damit die Verbindung zwischen dem Eingang des ersten Schaltelements und dem Ausgang des ersten Schaltelements und damit auch die Verbindung zwischen dem Elektrodeneingang der Schaltelementanordnung und dem Ausgang der Schaltelementanordnung. Dabei kann der Triggereingang beispielsweise mit dem Ausgang des ersten Schaltelements verbunden sein und so ausgebildet sein, dass ein Signal, dass zum Wechsel zwischen den beiden Zuständen führt, ein Spannungspuls ist, der im Abschaltbereich liegt. Damit würde das Flipflop genau dann seinen Zustand wechseln, wenn die Verbindung zwischen dem Elektrodeneingang der Schaltelementanordnung und dem Ausgang der Schaltelementanordnung getrennt werden soll. Die Verwendung einer Speicherschaltung in Form eines Flipflops hat den Vorteil, dass beispielsweise die Wiederinbetriebnahme eines Elektrodengürtels, in dem die aktive Schutzschaltung verbaut ist, verhindert wird, nachdem der Elektrodengürtel ein Mal einem Defibrillatorpuls ausgesetzt war. Damit wird verhindert, dass bei eventuell doch durch den Defibrillatorpuls erzeugten Schäden fehlerhafte Messungen durch den Impedanztomographen vorgenommen werden oder der Impedanztomograph durch eine beschädigte Schaltung ebenfalls beschädigt wird. Es ist allerdings bevorzugt, wenn beispielsweise ein Schalter vorgesehen ist, mit dem der Flipflop wieder in den ersten Zustand zurückversetzt werden kann, nachdem die aktive Schutzschaltung und die übrigen Komponenten des Elektrodengürtels auf ihre Integrität untersucht worden sind und keine Fehler festgestellt wurden.

In einer bevorzugten Ausführungsform umfasst die Schaltelementanordnung zumindest zwei Steuereingänge und ein zweites Schaltelement mit einem Eingang, einem Ausgang und einem Steuereingang. Ein erster Steuereingang der zumindest zwei Steuereingänge ist mit dem Steuereingang des ersten Schaltelements verbunden. Der Eingang des zweiten Schaltelements ist mit dem Elektrodeneingang der Schaltelementanordnung verbunden. Der Ausgang des zweiten Schaltelements ist mit dem Ausgang der Schaltelementanordnung verbunden. Der Steuereingang des zweiten Schaltelements ist mit einem zweiten Steuereingang der zumindest zwei Steuereingänge der Schaltelementanordnung verbunden. Das erste und das zweite Schaltelement sind in Serie geschaltet. Das zweite Schaltelement ist so ausgebildet, dass das zweite Schaltelement eine leitende Verbindung zwischen dem Eingang des zweiten Schaltelements und dem Ausgang des zweiten Schaltelements ausbildet, wenn eine an dem Steuereingang des zweiten Schaltelements anliegende Steuerspannung in einem dritten Spannungsbereich ist und das zweite Schaltelement keine leitende Verbindung zwischen dem Eingang des zweiten Schaltelements und dem Ausgang des zweiten Schaltelements ausbildet, wenn die an dem Steuereingang des zweiten Schaltelements anliegende Spannung in einem vierten Spannungsbereich ist. Die Schaltelementanordnung ist so ausgebildet, dass die an dem zumindest einen Steuereingang anliegende Spannung in den vierten Spannungsbereich wechselt, wenn an dem Elektrodeneingang eine Spannung anliegt, die einen vorbestimmten zweiten Schwellwert unterschreitet.

Mit anderen Worten umfasst die aktive Schutzschaltung in dieser bevorzugten Ausführungsform neben dem ersten Schaltelement ein zweites Schaltelement. Die beiden Schaltelemente sind in Serie geschaltet, d.h. der Eingang des einen Schaltelements ist mit dem Ausgang des anderen Schaltelements verbunden. Beispielsweise kann der Eingang des zweiten Schaltelements mit dem Elektrodeneingang verbunden sein, der Ausgang des zweiten Schaltelements ist mit dem Eingang des ersten Schaltelements verbunden sein und der Ausgang des ersten Schaltelements ist mit dem Ausgang der Schaltelementanordnung verbunden. Das zweite Schaltelement ist somit zwischen dem ersten Schaltelement und dem Elektrodeneingang angeordnet. Das zweite Schaltelement ist so ausgebildet, dass es eine leitende Verbindung zwischen dem Eingang des zweiten Schaltelements dem Ausgang des zweiten Schaltelements ausbildet, wenn die an dem Steuereingang des zweiten Schaltelements anliegende Spannung in einem dritten Bereich liegt, und den Eingang des zweiten Schaltelements vom Ausgang des zweiten Schaltelements trennt, wenn die Steuerspannung in einem vierten Bereich liegt. Der erste Spannungsbereich kann beispielsweise mit dem dritten Spannungsbereich übereinstimmen, es ist aber auch möglich, dass der erste Spannungsbereich beispielsweise zwischen + 5 V und + 10 V liegt und der dritte Spannungsbereich zwischen - 10 V und - 5 V liegt. Der zweite Spannungsbereich könnte auch mit dem vierten Spannungsbereich übereinstimmen. Es ist allerdings auch denkbar, dass der zweite Spannungsbereich sämtliche Spannungen kleiner 5 V umfasst und der vierte Spannungsbereich sämtliche Spannungen größer - 5 V umfasst. In jedem Falle ist die Schaltelementanordnung so ausgelegt, dass die an dem Steuereingang des zweiten Schaltelements anliegende Spannung in den vierten Spannungsbereich wechselt, d.h. das zweite Schaltelement leitet nicht mehr, wenn an dem Elektrodeneingang eine Spannung anliegt, die einen vorbestimmten zweiten Schwellwert unterschreitet. Der vorbestimmte zweite Schwellwert kann beispielsweise einen zweiten Abschnitt des Abschaltbereichs nach oben hin begrenzen. Durch die Verwendung eines derartigen zweiten Schaltelements wird auf vorteilhafte Weise sichergestellt, dass die aktive Schutzschaltung sowohl bei negativen als auch bei positiven Spannungspulsen abschaltet.

Dabei ist es bevorzugt, wenn das zweite Schaltelement ein normal sperrender p-Kanal MOSFET mit einem Drain-Anschluss, einem Source-Anschluss und einem Gate-Anschluss ist. Der Drain-Anschluss bildet den Eingang des zweiten Schaltelements, der Source-Anschluss bildet den Ausgang des zweiten Schaltelements und der Gate-Anschluss bildet den Steuereingang des zweiten Schaltelements. Die Verwendung eines normal sperrenden p-Kanal MOSFET hat den Vorteil, dass dieser bei positiven Steuerspannungen nicht leitend ist. Im Übrigen hat die Verwendung eines p-Kanal MOSFET die gleichen Vorteile, wie die Verwendung eines n-Kanal MOSFET als erstes Schaltelement. Durch die Kombination eines ersten Schaltelements in Form eines n-Kanal MOSFET mit einem zweiten Schaltelement in Form eines p-Kanal MOSFET wird sichergestellt, dass die aktive Schutzschaltung die Verbindung zwischen der Elektrode und dem nachgeordneten Messverstärker auch bei einem bipolaren Defibrillatorpulse zuverlässig trennt.

Die Erfindung wird nachfolgend anhand von Zeichnungen, die verschiedene Ausführungsbeispiele von aktiven Schutzschaltungen und ein Ausführungsbeispiel eines erfindungsgemäßen Elektrodengürtels zeigen, näher erläutert, wobei
- Fig. 1: ein erstes Ausführungsbeispiel einer erfindungsgemäßen aktiven Schutzschaltung zeigt,
- Fig. 2: ein zweites Ausführungsbeispiel einer erfindungsgemäßen aktiven Schutzschaltung zeigt,
- Fig. 3: ein drittes Ausführungsbeispiel einer erfindungsgemäßen aktiven Schutzschaltung zeigt,
- Fig. 4: ein erstes Ausführungsbeispiel zweier miteinander verschalteter erfindungsgemäßer aktiver Schutzschaltungen zeigt,
- Fig. 5: ein zweites Ausführungsbeispiel zweier miteinander verschalteter erfindungsgemäßer aktiver Schutzschaltungen zeigt und
- Fig. 6: eine schematische Darstellung eines Ausführungsbeispiels eines erfindungsgemäßen Elektrodengürtels zeigt.

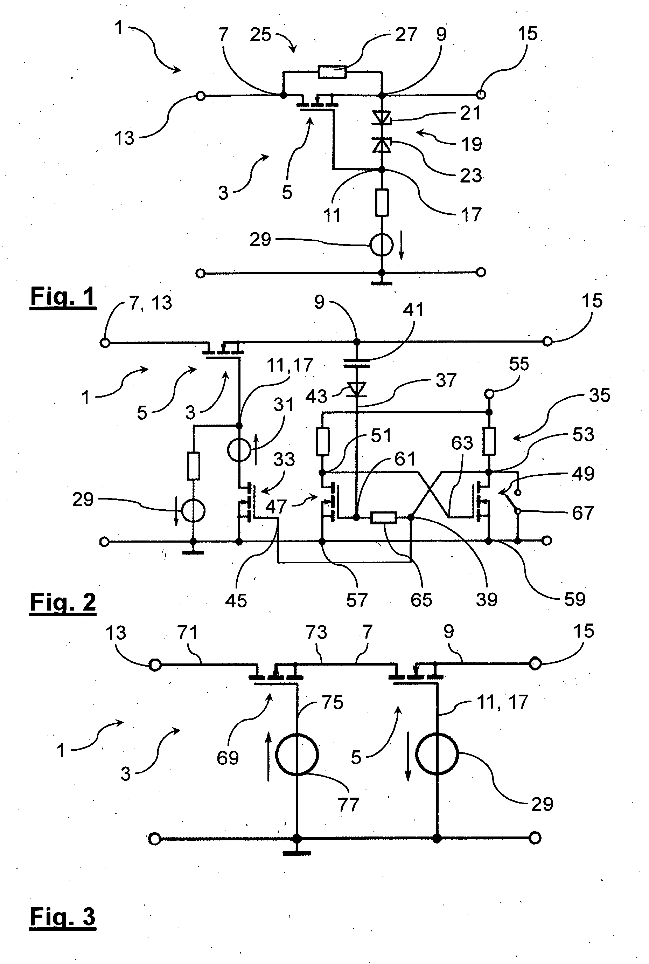

In der nachfolgenden Beschreibung der Ausführungsbeispiele aus den Figuren 1 bis 5 werden gleich bezeichnete Elemente mit den gleichen Bezugszeichen versehen.

Fig. 1 zeigt ein erstes Ausführungsbeispiel einer erfindungsgemäßen aktiven Schutzschaltung 1 mit einer Schaltelementanordnung 3. Die Schaltelementanordnung 3 umfasst ein erstes Schaltelement 5 in Form eines normal sperrenden n-Kanal MOSFET 5. Der n-Kanal MOSFET 5 weist einen Drain-Anschluss 7 auf, der gleichzeitig den Eingang 7 des ersten Schaltelements 5 bildet. Weiterhin weist der n-Kanal MOSFET 5 einen Source-Anschluss 9 auf, der den Ausgang 9 des ersten Schaltelements 5 bildet. Schließlich umfasst der n-Kanal MOSFET 5 noch einen Gate-Anschluss 11, der den Steuereingang 11 des ersten Schaltelements 5 bildet.

Der Eingang 7 des ersten Schaltelements 5 ist mit dem Elektrodeneingang 13 der Schaltelementanordnung 3 verbunden. Der Elektrodeneingang 13 ist zur Verbindung mit einer Elektrode eines Elektrodengürtels für einen elektrischen Impedanztomographen geeignet. Der Ausgang 9 des ersten Schaltelements 5 ist mit dem Ausgang 15 der Schaltelementanordnung 3 verbunden. Der Ausgang 9 ist zur Verbindung mit einem Messverstärker eines elektrischen Impedanztomographen geeignet. Der Messverstärker kann beispielsweise zusammen mit der aktiven Schutzschaltung 1 in dem Elektrodengürtel angeordnet sein. Dies hat den Vorteil, dass die von der mit dem Elektrodeneingang verbundenen Elektrode erfassten Spannungen lokal im Elektrodengürtels verstärkt werden können und die Übertragung des Messsignals an den Impedanztomographen weniger fehleranfällig ist und noch kleinere Spannungen übertragen werden können. Es ist allerdings auch denkbar, dass die aktive Schutzschaltung 1 in dem Elektrodengürtel angeordnet ist und der Messverstärker in dem eigentlichen Impedanztomographen. Der Steuereingang 11 des ersten Schaltelements 5 bildet gleichzeitig den ersten Steuereingang 17 der Schaltelementanordnung 3.

Die in Fig. 1 dargestellte aktive Schutzschaltung 1 umfasst weiterhin ein Sperrelement 19 aus zwei gegeneinander geschalteten Z-Dioden 21, 23. Das Sperrelement 19 verbindet den Ausgang 9 des ersten Schaltelements 5 mit dem Steuereingang 11 des ersten Schaltelements 5. Die beiden Z-Dioden 21, 23 weisen jeweils auf bekannte Weise eine Sperrrichtung und eine Durchlassrichtung auf, wobei eine erste Z-Diode 21 der beiden Z-Dioden 21, 23 so geschaltet ist, dass ihre Durchlassrichtung der Durchlassrichtung einer zweiten Z-Diode 23 der beiden Z-Dioden 21, 23 entgegengesetzt ist.

Weiterhin umfasst die aktive Schutzschaltung 1 eine Bypassschaltung 25 die von einem hochohmigen Widerstand 27 gebildet wird. Die Bypassschaltung 25 verbindet den Eingang 7 des ersten Schaltelements 5 mit dem Ausgang 9 des ersten Schaltelements 5. Schließlich ist noch eine Spannungsquelle 29 vorgesehen, über die an dem Steuereingang 11 des ersten Schaltelements 5 eine Betriebsspannung von beispielsweise 5 V angelegt werden kann. Die Spannungsquelle 29 ist einseitig auf Masse gelegt und kann beispielsweise in Form einer Batterie bereitgestellt werden oder direkt von einem Impedanztomographen gespeist werden.

Die aktive Schutzschaltung 1 umfasst ein erstes Schaltelement 5 in Form eines normal sperrenden n-Kanal MOSFET 5. Durch die Verwendung des normal sperrenden n-Kanal MOSFET 5 ist die aktive Schutzschaltung 1 auf vorteilhafte Weise eigensicher, da der Elektrodeneingang 13 von dem Ausgang 15 der Schaltelementanordnung 3 getrennt wird, wenn aktive Schutzschaltung 1 spannungslos ist.

Wird im normalen Messbetrieb über die Spannungsquelle 29 eine Betriebsspannung in einem ersten Spannungsbereich an dem Steuereingang 11 angelegt, so stellt das erste Schaltelement 5 eine leitende Verbindung zwischen dem Eingang 7 des ersten Schaltelements und dem Ausgang 9 des ersten Schaltelements bereit. Damit wird auch der Elektrodeneingang 13 mit dem Ausgang 15 der Schaltelementanordnung 3 leitend verbunden und eventuell von der Elektrode aufgenommene Messsignale werden an einen nachgeordneten Messverstärker weitergegeben. Umgekehrt werden auch über den Ausgang 15 an die aktive Schutzschaltung 1 angelegt Ströme, die für die elektrische Impedanztomographie benötigt werden, an den Elektrodeneingang zur Einspeisung weitergeleitet. Die erste Z-Diode 21 verhindert, dass die von der Spannungsquelle 29 bereitgestellte Betriebsspannung an dem Ausgang 9 des ersten Schaltelements 5 anliegt und das weitergeleitete Messsignal verfälscht. Die beiden entgegengesetzt in Serie geschalteten Z-Dioden 21, 23 begrenzen zudem die Gate-Source-Spannung des MOSFET 5 in beiden Richtungen.

Die aktive Schutzschaltung 1 ist so ausgestaltet, dass beim Anlegen einer Spannung an dem Elektrodeneingang 13 und damit auch an dem Ausgang 9 des ersten Schaltelements 5, die in einem Abschaltbereich liegt, genauer gesagt, über einem vorgegebenen Schwellwert liegt, die an dem Steuereingang 11 des ersten Schaltelements 5 anliegende Steuerspannung nicht mehr ausreicht, um das erste Schaltelement 5 leitend zu schalten. Das erste Schaltelement 5 trennt sodann die Verbindung zwischen dem Eingang 7 und dem Ausgang 9. Durch die Verwendung der zweiten Z-Diode 23 wird sichergestellt, dass die Spannung, die zwischen dem Ausgang 9 des ersten Schaltelements und dem Steuereingang 11 anliegt, auf einen maximalen Wert begrenzt ist. Damit ist der der aktiven Schutzschaltung 1 nachgeordnete Messverstärker vor einem an dem Elektrodeneingang 13 anliegenden Spannungspuls sicher.

Durch die Verwendung der Bypassschaltung 25 wird auf vorteilhafte Weise sichergestellt, dass auch nach dem Trennen der leitenden Verbindung zwischen dem Eingang 7 und dem Ausgang 9 des ersten Schaltelements 5 noch eine hinreichend hohe Spannung am Ausgang 9 anliegt, so dass das erste Schaltelement 5 die Verbindung weiter getrennt hält, solange die an dem Elektrodeneingang 13 anliegende Spannung in einem Hochvoltbereich ist. Fällt die Spannung so weit ab, dass die zweite Z-Diode 23 nicht mehr leitet und die Spannungsdifferenz zwischen dem Steuereingang 11 des Schaltelements 5 und dessen Ausgang 9 wieder in den ersten Spannungsbereich wechselt, dann reicht die von der Spannungsquelle 29 am Steuereingang 11 des ersten Schaltelements 5 angelegte Spannung aus, um das Schaltelement 5 leitend zu schalten. D.h., das Schaltelement verbindet wieder den Eingang 7 des ersten Schaltelements 5 leitend mit dem Ausgang 9 des ersten Schaltelements 5.

Fig. 2 zeigt ein zweites Ausführungsbeispiel einer erfindungsgemäßen aktiven Schutzschaltung 1 mit einer Schaltelementanordnung 3, die ein erstes Schaltelement 5 in Form eines normal sperrenden n-Kanal MOSFET 5 umfasst. Wie in dem in Fig. 1 gezeigten ersten Ausführungsbeispiel weist der n-Kanal MOSFET 5 einen Drain-Anschluss 7 auf, der den Eingang 7 des ersten Schaltelements 5 bildet und mit dem Elektrodeneingang 13 der Schaltelementanordnung 3 verbunden ist. Weiterhin umfasst der n-Kanal MOSFET 5 einen Source-Anschluss 9, der den Ausgang 9 des ersten Schaltelements 5 bildet und mit dem Ausgang 15 der Schaltelementanordnung 3 verbunden ist. Schließlich umfasst der n-Kanal MOSFET 5 noch einen Gate-Anschluss 11, der den Steuereingang 11 des ersten Schaltelements 5 und gleichzeitig den ersten Steuereingang 17 der Schaltelementanordnung 3 bildet. Analog zum ersten Ausführungsbeispiel umfasst die aktive Schutzschaltung 1 in Fig. 2 eine Spannungsquelle 29, über die eine Steuerspannung im ersten Spannungsbereich an den Steuereingang 11 des ersten Schaltelements 5 angelegt werden kann. Die Spannungsquelle 29 ist einseitig auf Masse gelegt.

Das zweite Ausführungsbeispiel einer erfinderischen Schutzschaltung 1 umfasst zudem eine zweite Spannungsquelle 31, die ebenfalls mit dem Steuereingang 11 des ersten Schaltelements 5 verbunden ist. Die zweite Spannungsquelle 31 ist über ein Schaltelement 33 in Form eines normal sperrenden n-Kanal MOSFET 33 auf Masse gelegt, d.h., die zweite Spannungsquelle 31 ist nur aktiv, wenn der n-Kanal MOSFET 33 leitend ist. In diesem Fall liegen sowohl die Spannungsquelle 29 als auch die zweite Spannungsquelle 31 am Steuereingang 11 des ersten Schaltelements 5 an. Die von der zweiten Spannungsquelle 31 bereitgestellte Spannung wird so gewählt, dass die Summe aus den von den beiden Spannungsquellen 29, 31 bereitgestellten Spannungen im zweiten Spannungsbereich liegt, in dem das erste Schaltelement 5 keine leitende Verbindung zwischen dem Eingang 7 und dem Ausgang 9 des ersten Schaltelements 5 und damit auch zwischen dem Elektrodeneingang 13 und dem Ausgang 15 der Schaltelementanordnung 3 ausbildet.

Die Steuerung des Schaltelements 33 erfolgt mittels einer Speicherschaltung 35 in Form eines Flipflops 35. Die Speicherschaltung 35 umfasst einen Triggereingang 37 und einen Schaltausgang 39. Der Triggereingang 37 ist über einen Kondensator 41 und eine Diode 43 mit dem Ausgang 9 des ersten Schaltelements 5 verbunden; der Schaltausgang 39 ist mit einem Gate-Anschluss 45 des n-Kanal MOSFET 33, der das Schaltelement 33 bildet, verbunden. Die Speicherschaltung 35 ist so ausgebildet, dass an dem Schaltausgang 39 und damit auch an dem Gate-Anschluss 45 des Schaltelementes 33 eine Spannung in einem zweiten Spannungsbereich anliegt, in dem der n-Kanal MOSFET 33 nicht leitend ist und die zweite Spannungsquelle 31 damit nicht aktiv ist, wenn die aktive Schutzschaltung 1 im normalen Messbetrieb betrieben wird. Die Speicherschaltung 35 ist dann in einem ersten Zustand. Empfängt die Speicherschaltung 35 an dem Triggereingang 37 einen Triggerpuls, der beispielsweise in der ansteigenden Flanke eines Spannungspulses eines Defibrillators bestehen kann, die im Abschaltbereich liegt, dann wechselt die Speicherschaltung 35 aus dem ersten Zustand in einen zweiten Zustand, in dem an dem Schaltausgang 39 und damit an dem Gate-Anschluss 45 des Schaltelements 33 eine Spannung in einem zweiten Spannungsbereich anliegt, in dem das Schaltelement 33 leitend ist und die zweite Spannungsquelle 31 mit Masse verbunden und damit aktiv wird. In diesem Fall trennt das erste Schaltelement 5 die Verbindung zwischen dem Eingang 7 und dem Ausgang 9 des ersten Schaltelements 5. Damit werden dem Ausgang 15 der Schaltelementanordnung 3 nachgeordnete Elemente eines Impedanztomographen vor möglichen Spannungsstößen geschützt. Die Speicherschaltung 35 verhindert auf vorteilhafte Weise, dass die Schaltelementanordnung 3 nach einmaligem Anlegen eines Spannungspulses in einem Abschaltbereich wieder ohne aktive Handlungen in Betrieb genommen werden kann.

Die eigentliche Speicherschaltung 35 umfasst ein erstes Speicherschaltelement 47 und ein zweites Speicherschaltelement 49. Die beiden Speicherschaltelemente 47, 49 werden von normal sperrenden n-Kanal MOSFET 47, 49 gebildet. Beide Speicherschaltelemente 47, 49 weisen einen Drain-Anschluss 51, 53 auf, die jeweils mit einer Betriebsspannungsquelle 55 verbunden sind. Weiterhin weist jedes der Speicherschaltelemente 47, 49 einen Source-Anschluss 57, 59 auf, die auf Masse liegen. Die Masse kann beispielsweise durch die Masse des Impedanztomographen gebildet werden. Schließlich weist jedes der Speicherschaltelemente 47, 49 noch einen Gate-Anschluss 61, 63 auf. Der Gate-Anschluss 61 des ersten Speicherschaltelements 47 ist über einen Widerstand 65 mit dem Drain-Anschluss 53 des zweiten Speicherschaltelements 49 verbunden. Der Gate-Anschluss 63 des zweiten Speicherschaltelements 49 ist direkt mit dem Drain-Anschluss 51 des ersten Speicherschaltelements 47 verbunden. Der Triggereingang 37 und der Schaltausgang 39 sind beide zwischen den Gate-Anschluss 61 des ersten Speicherschaltelements 47 und den Drain-Anschluss 53 des zweiten Speicherschaltelement 49 geschaltet, wobei der Triggereingang 37 direkt auf den Gate-Anschluss 61 des ersten Speicherschaltelements 47 gelegt ist und zwischen dem Triggereingang 37 und dem Schaltausgang 39 der Widerstand 65 angeordnet ist.

Wird die aktive Schutzschaltung 1, die in Fig. 2 dargestellt ist, in Betrieb genommen, dann ist zunächst das zweite Speicherschaltelement 49 leitend geschaltet, während das erste Speicherschaltelement 47 nicht leitend ist. Damit fällt die am Drain-Anschluss 53 des zweiten Speicherschaltelements 49 von der Betriebsspannungsquelle 55 bereitgestellte Spannung direkt über das zweite Speicherschaltelement 49 auf Masse ab. An dem Schaltausgang 39 liegt somit keine hinreichend hohe Spannung an, um das Schaltelement 33 leitend zu schalten. Wird über den Triggereingang 37 ein Triggersignal empfangen, so wird das erste Speicherschaltelement 47 leitend geschaltet und an dem Gate-Anschluss 63 des zweiten Speicherschaltelements 49 liegt keine hinreichend hohe Spannung mehr an, um dieses weiter leitend zu halten. Daher sperrt das zweite Speicherschaltelement 49 die Verbindung zwischen dem Drain-Anschluss 53 und dem Source-Anschluss 59. Dies führt dazu, dass die Spannung am Gate-Anschluss 61 des ersten Speicherelements 47 dauerhaft soweit ansteigt, dass das erste Speicherschaltelement 47 leitend gehalten wird. Gleichzeitig liegt am Schaltausgang 39 und damit auch am Gate-Anschluss 45 des Schaltelements 33 eine hinreichend hohe Spannung an, um das Schaltelement 33 leitend zu schalten. Damit wechselt das erste Schaltelement 5 in einen zweiten Zustand, in dem die Verbindung zwischen dem Eingang 7 und dem Ausgang 9 des ersten Schaltelements 5 getrennt ist. Damit wird auf vorteilhafte Weise verhindert, dass die aktive Schutzschaltung 1 wieder in Betrieb genommen wird, nachdem sie einmal aufgrund einer Überspannung deaktiviert worden ist.

Um die aktive Schutzschaltung 1 doch weiter zu verwenden, nachdem die Funktionsfähigkeit der aktiven Schutzschaltung 1 und der nachgeordneten Komponenten sowie der vorgeordneten Elektrode überprüft worden ist, ist ein Schalter 67 vorgesehen. Wird der Schalter 67 geschlossen, fällt die am Drain-Anschluss 53 des zweiten Speicherschaltelements 49 anliegende Spannung direkt auf Masse ab. Damit steht am Gate-Eingang 61 des ersten Speicherschaltelements 47 keine ausreichend hohe Spannung mehr bereit, um das erste Speicherschaltelement 47 leitend zu halten. Damit steigt aber die Spannung am Gate-Anschluss 63 des zweiten Speicherschaltelements 49 wieder so weit an, dass das zweite Speicherschaltelement 49 wieder leitend geschaltet wird und die Speicherschaltung 35 wieder in den ersten Zustand wechselt. Im ersten Zustand liegt am Schaltausgang 39 keine ausreichend hohe Spannung an, um das Schaltelement 33 leitend zu schalten. Damit ist die am Steuereingang 11 des ersten Schaltelements 5 anliegende Spannung wieder im ersten Bereich und das erste Schaltelement 5 bildet eine leitende Verbindung zwischen dem Eingang 7 und dem Ausgang 9 des ersten Schaltelements 5 aus. Der Schalter 67 ermöglicht damit auf vorteilhafte Weise, die Speicherschaltung 35 zurückzusetzen.

Ein drittes Ausführungsbeispiel einer aktiven Schutzschaltung 1 ist in Fig. 3 schematisch dargestellt. Diese Schaltung verdeutlicht lediglich, dass ein Schutz vor bipolaren Spannungspulsen, die an einem Elektrodeneingang 13 anliegen, durch eine Schaltelementanordnung 3 mit einer Reihenschaltung eines ersten Schaltelements 5 in Form eines normal sperrenden n-Kanal MOSFET 5 und eines zweiten Schaltelements 69 in Form eines normal sperrenden p-Kanal MOSFET 69 erreicht werden kann. Das zweite Schaltelement 69 weist ebenfalls einen Eingang 71, einen Ausgang 73 und einen Steuereingang 75 auf. Der Eingang 71 des zweiten Schaltelements 69 wird von dem Drain-Anschluss 71 des p-Kanal MOSFET 69 gebildet. Der Ausgang 73 des zweiten Schaltelements 69 wird von dem Source-Anschluss 73 des p-Kanal MOSFET 69 gebildet. Der Steuereingang 75 des zweiten Schaltelements 69, der gleichzeitig ein zweiter Steuereingang 75 der Schaltelementanordnung 3 ist, wird von dem Gate-Anschluss 75 des p-Kanal MOSFET 69. Zudem weist die aktive Schutzschaltung 1 eine weitere Spannungsquelle 77 auf, über die an den zweiten Steuereingang 75 eine Betriebsspannung in einem dritten Bereich angelegt werden kann, in dem das zweite Schaltelement 69 leitet.

Der Aufbau des ersten Schaltelements 5 entspricht dem, der bereits in Bezug auf die in den Figuren 1 und 2 gezeigten Ausführungsbeispiele näher beschrieben worden ist. Die in Fig. 3 gezeigte Ausführungsform eines aktiven Schutzschaltung 1 hat den Vorteil, dass sie sowohl bei einem negativen Spannungspuls am Elektrodeneingang 13 als auch bei einem positiven Spannungspuls am Elektrodeneingang 13 die Verbindung zwischen dem Elektrodeneingang 13 und dem Ausgang 15 der Schaltelementanordnung 3 trennt. Dabei ist das zweite Schaltelement 69 so ausgestaltet, dass es die Verbindung zwischen dem Eingang 71 des Schaltelements 69 und dem Ausgang 73 des Schaltelements 69 trennt, wenn die am Elektrodeneingang 13 anliegende Spannung einen zweiten vorbestimmten Schwellwert unterschreitet, während das erste Schaltelement 5 so ausgebildet ist, dass es die Verbindung zwischen dem Eingang 7 und dem Ausgang 9 des ersten Schaltelements 5 trennt, wenn die an dem Elektrodeneingang 13 anliegende Spannung einen ersten vorbestimmten Schwellwert überschreitet.

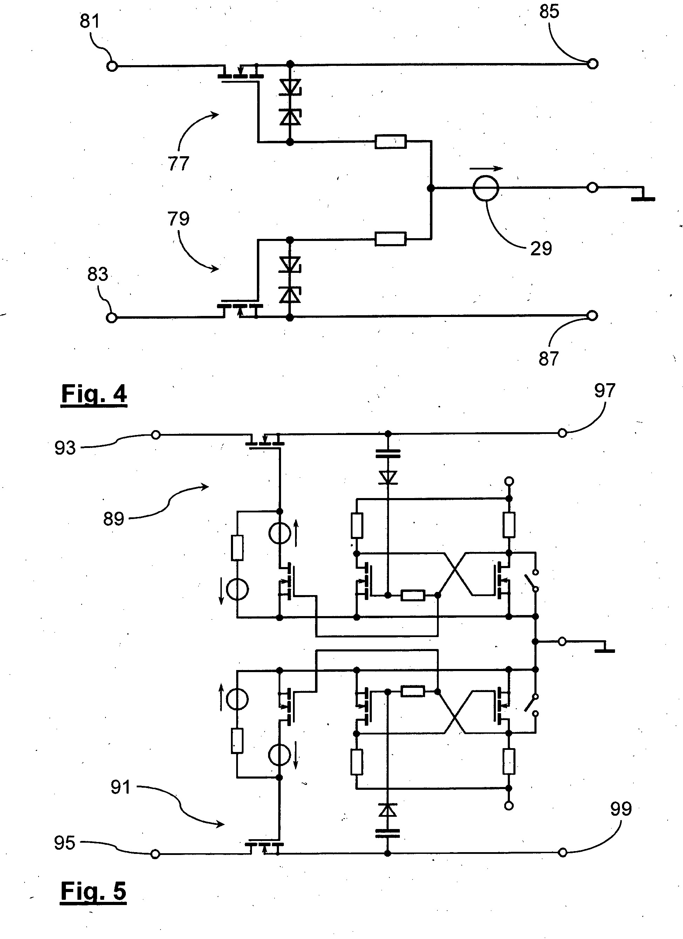

In Fig. 4 ist eine Verschaltung zweier aktiver Schutzschaltungen 77, 79 dargestellt, wie sie sich beim Betrieb eines elektrischen Impedanztomographen ergibt. Im Betrieb eines elektrischen Impedanztomographen werden stets zwei Elektrodeneingänge 81, 83 miteinander kombiniert, um einen Strom einzuspeisen oder die Spannung, die zwischen diesen beiden Elektrodeneingängen 81, 83 abfällt, zu messen. Die beiden aktiven Schutzschaltung 77, 79 entsprechen der aktiven Schutzschaltungen 1, die in Fig. 1 dargestellt ist. Daher wird hier auf die Details der Schutzschaltungen 77, 79 nicht weiter eingegangen. Die Schutzschaltungen 77, 79 unterscheiden sich von der in Fig. 1 dargestellten aktiven Schutzschaltung 1 lediglich darin, dass auf eine Bypassschaltung 25 verzichtet worden ist, da diese nicht zwingend notwendig ist. Beide Schutzschaltungen 77, 79 werden über eine gemeinsame Spannungsquelle 29 betrieben, die beispielsweise direkt von dem Impedanztomographen bereitgestellt wird und die mit der Masse des Impedanztomographen verbunden ist.

Nicht dargestellt ist in Fig. 4, dass die Ausgänge 85, 87 im Impedanztomographen über eine leitende Verbindung mit einander verbunden sind. Um zu verhindern, dass der Defibrillatorpuls sich durch die Elektrodeneingang 81, 83 und die Ausgänge 85 87 in die nachgeordneten Messverstärker und den nachgeordneten Impedanztomographen ausbreiten kann, genügt es daher wenn eine der beiden aktiven Schutzschaltungen 70, 79 die Verbindung zwischen dem ihr zugeordneten Elektrodeneingang 81, 83 und dem ihr zugeordneten Ausgang 85, 87 auftrennt.

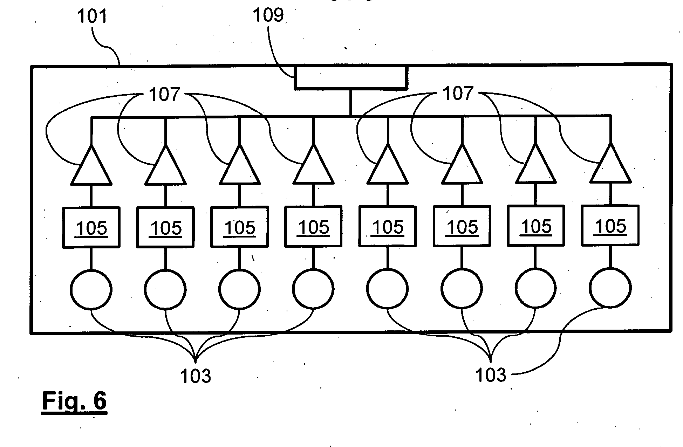

In Fig. 5 ist, analog zu der Ausgestaltung in Fig. 4, eine Kombination zweier aktiver Schutzschaltungen 89, 91 dargestellt, bei dem die Schutzschaltungen 89, 91 dem in Fig. 2 ausführlich dargestellten Ausführungsbeispiel entsprechen. Daher wird auch hier auf eine detailliertere Beschreibung der Schaltungen verzichtet. Wie in Fig. 4 sind die beiden mit den Elektrodeneingänge 93, 95 verbundenen Elektroden über die Ausgänge 97, 99 innerhalb des Impedanztomographen, beispielsweise über einen Messverstärker, leitend miteinander verbunden. Am Patienten sind die beiden verbundenen Elektroden an verschiedenen Stellen angeordnet und liegen bei einem Defibrillatorpuls auf verschiedenen Potentialen. Daher genügt es wiederum, dass beim Anlegen eines Defibrillatorpulses an einen Patienten, an dem die mit den Elektrodeneingängen 93, 94 verbundenen Elektroden angelegt sind, eine der beiden Schutzschaltungen 89, 91 die Verbindung zwischen dem jeweiligen Elektrodeneingang 93, 95 und dem jeweiligen Ausgang 97, 99 trennt, um die den aktiven Schutzschaltungen 89, 91 nachgeordneten elektronischen Komponenten vor dem Defibrillatorpuls zu schützen und um zu verhindern, dass zu viel Energie des Defibrillatorpulses in den Impedanztomographen abfließt. Da auch bei einem bipolaren Defibrillatorpuls an einem der beiden Elektrodeneingänge 93, 95 ein positiver Spannungspuls anliegen wird, wird eine der beiden aktiven Schutzschaltung 89, 91 in jedem Falle die Verbindung zwischen Ihrem Elektrodeneingang 93, 95 und ihrem Ausgang 97, 99 trennen. Die in Figuren 4 und 5 dargestellten Anordnungen sind damit auf vorteilhafte Weise auch ohne die Verwendung einer Reihenschaltung aus einem n-Kanal MOSFET und einem p-Kanal MOSFET bei bipolaren Defibrillatorpulsen sicher. Schließlich zeigt Fig. 6 eine schematische Darstellung eines Ausführungsbeispiels eines erfindungsgemäßen Elektrodengürtels 101 für einen elektrischen Impedanztomographen. Der Elektrodengürtel 101 umfasst eine Mehrzahl von Elektroden 103, die zur Anordnung auf der Haut eines Patienten vorgesehen sind. Jede der Elektroden 103 ist über eine erfindungsgemäße aktive Schutzschaltung 105 mit einem aktiven Messverstärker 107 verbunden, der ebenfalls auf dem Elektrodengürtel 101 angeordnet ist. Bei den aktiven Schutzschaltungen 105 kann es sich um Schutzschaltungen gemäß einem der vorhergehenden Ausführungsbeispiele handeln. Dabei sind die Elektrodeneingänge der aktiven Schutzschaltungen 105 jeweils mit den Elektroden verbunden und die Ausgänge mit den nachgeordneten aktiven Messverstärkern 107. Die aktiven Messverstärker sind über nur schematisch dargestellte Verbindungen mit einem Ausgang 109 verbunden, über den die Elektroden 103 schließlich mit dem eigentlichen Impedanztomographen verbunden werden können. Die Vorteile des Elektrodengürtels 101 entsprechen den Vorteilen der darin verwendeten aktiven Schutzschaltungen 105. Zudem ermöglicht die Anordnung der aktiven Messverstärker 107 direkt im Elektrodengürtel 101 eine aktive Verstärkung der gemessenen Spannung nah an der Elektrode 103, womit die Empfindlichkeit des Impedanztomographen gegenüber einer erstmaligen Verstärkung im eigentlichen Impedanztomographen erhöht werden kann.

## Patentansprüche

1. Elektrodengürtel (101) für einen elektrischen Impedanztomographen mit:
einer Mehrzahl von Elektroden (103), die zur Anordnung auf der Haut eines Patienten vorgesehen sind,
einer Mehrzahl von Messverstärkern (107), und
einer Mehrzahl von aktiven Schutzschaltungen (1, 77, 79, 89, 91) für jeden der Mehrzahl von Messverstärkern,
wobei jeder der Mehrzahl von Elektroden zumindest eine der Mehrzahl von aktiven Schutzschaltungen zugeordnet ist und jede der Mehrzahl von aktiven Schutzschaltungen nur einer der Mehrzahl von Elektroden zugeordnet ist,
wobei jede der Mehrzahl von Elektroden (103) über zumindest eine der Mehrzahl von aktiven Schutzschaltungen mit einem der Mehrzahl von Messverstärkern verbunden ist,
wobei jede der Mehrzahl von aktiven Schutzschaltungen eine Schaltelementanordnung (3) aufweist,
wobei die Schaltelementanordnung (3) einen Elektrodeneingang (13) und einen Ausgang (15) und zumindest einen Steuereingang (17, 75) für eine Steuerspannung aufweist,
wobei der Elektrodeneingang (13) der Schaltelementanordnung (3) mit einer der Mehrzahl von Elektroden (103) des Elektrodengürtels (101) verbunden ist,
wobei der Ausgang (15) der Schaltelementanordnung (3) mit einem der Mehrzahl von Messverstärkern (107) verbunden ist,
wobei die Schaltelementanordnung (3) derart ausgebildet ist, dass die Schaltelementanordnung (3) eine leitende Verbindung zwischen dem Elektrodeneingang (13) der Schaltelementanordnung (3) und dem Ausgang (15) der Schaltelementanordnung (3) ausbildet, wenn eine an dem zumindest einen Steuereingang (17, 75) anliegende Steuerspannung in einem ersten Spannungsbereich ist, und die Schaltelementanordnung (3) keine leitende Verbindung zwischen dem Elektrodeneingang (13) der Schaltelementanordnung (3) und dem Ausgang (15) der Schaltelementanordnung (3) ausbildet, wenn die an dem zumindest einen Steuereingang (17, 75) anliegende Steuerspannung in einem zweiten Spannungsbereich ist, und
wobei jede der Mehrzahl von aktiven Schutzschaltungen (1, 77, 79, 89, 91) so ausgebildet ist, dass die an dem zumindest einen Steuereingang (17, 75) anliegende Spannung in dem zweiten Spannungsbereich ist, wenn an dem Elektrodeneingang (13) eine Spannung anliegt, die in einem Abschaltbereich liegt.

2. Aktive Schutzschaltung (1, 77, 79) für einen Elektrodengürtel gemäß Anspruch 1, wobei der Ausgang (15) der Schaltelementanordnung (3) mit dem zumindest einen Steuereingang (17, 75) der Schaltelementanordnung über ein Sperrelement (19) verbunden ist, wobei das Sperrelement (19) so ausgebildet ist, dass die an dem Ausgang (15) der Schaltelementanordnung (3) anliegende Spannung nur dann an den Steuereingang (17, 75) angelegt wird, wenn die am Ausgang (15) der Schaltelementanordnung (3) anliegende Spannung in dem Abschaltbereich ist.

3. Aktive Schutzschaltung (1) für einen Elektrodengürtel nach Anspruch 2, wobei die aktive Schutzschaltung (1) parallel zu der Schaltelementanordnung (3) eine Bypassschaltung (25) aufweist, die den Eingang (13) der Schaltelementanordnung (3) mit dem Ausgang (15) der Schaltelementanordnung (3) verbindet, wobei die Bypassschaltung (25) so ausgebildet ist, dass an dem Ausgang (15) der Schaltelementanordnung (3) eine Spannung im Abschaltbereich anliegt, wenn die Schaltelementanordnung (3) keine leitende Verbindung zwischen dem Elektrodeneingang (13) der Schaltelementanordnung (3) und dem Ausgang (15) der Schaltelementanordnung (3) ausbildet, und die am Elektrodeneingang (13) der Schaltelementanordnung (3) anliegende Spannung in einem Hochvoltbereich ist, wobei der Hochvoltbereich ein Teilbereich des Abschaltbereichs ist.

4. Aktive Schutzschaltung (1, 89, 91) für einen Elektrodengürtel nach einem der vorhergehenden Ansprüche mit einer Speicherschaltung (35), die so ausgebildet ist, dass an dem zumindest einen Steuereingang (17, 75) dauerhaft eine Spannung angelegt wird, die im zweiten Spannungsbereich ist, wenn an dem Elektrodeneingang (13) einmal eine Spannung anliegt, die im Abschaltbereich ist.

5. Aktive Schutzschaltung (1, 77, 79, 89, 91) für einen Elektrodengürtel gemäß einem der vorhergehenden Ansprüche, wobei die Schaltelementanordnung (3), ein erstes Schaltelement (5) mit einem Eingang (7), einem Ausgang (9) und einem Steuereingang (11) aufweist,
wobei der Eingang (7) des ersten Schaltelements (5) mit dem Elektrodeneingang (13) der Schaltelementanordnung (3) verbunden ist, der Ausgang (9) des ersten Schaltelements (5) mit dem Ausgang (15) der Schaltelementanordnung (3) verbunden ist und der Steuereingang (11) des ersten Schaltelements (5) mit dem zumindest einen Steuereingang (17, 75) der Schaltelementanordnung (3) verbunden ist,
wobei das erste Schaltelement (5) so ausgebildet ist, dass das erste Schaltelement (5) eine leitende Verbindung zwischen dem Eingang (7) des ersten Schaltelements (5) und dem Ausgang (9) des ersten Schaltelements (5) ausbildet, wenn eine an dem Steuereingang (11) des ersten Schaltelements (5) anliegende Steuerspannung in dem ersten Spannungsbereich ist, und das erste Schaltelement (5) keine leitende Verbindung zwischen dem Eingang (7) des ersten Schaltelements (5) und dem Ausgang (9) des ersten Schaltelements (5) ausbildet, wenn die an dem Steuereingang (11) des ersten Schaltelements (5) anliegende Steuerspannung in dem zweiten Spannungsbereich ist,
wobei die aktive Schutzschaltung (1, 77, 79, 89, 91) so ausgebildet ist, dass die an dem zumindest einen Steuereingang (17, 75) anliegende Spannung in den zweiten Spannungsbereich wechselt, wenn an dem Elektrodeneingang (13) eine Spannung anliegt, die einen vorbestimmten ersten Schwellwert überschreitet.

6. Aktive Schutzschaltung (1, 77, 79, 89, 91) für einen Elektrodengürtel gemäß Anspruch 5, wobei das erste Schaltelement (5) ein normal sperrender n-Kanal MOSFET mit einem Drain-Anschluss (7), einem Source-Anschluss (9) und einem Gate-Anschluss (11) ist, wobei der Drain-Anschluss (7) den Eingang (7) des ersten Schaltelements (5) bildet, der Source-Anschluss (9) den Ausgang (9) des ersten Schaltelements (5) bildet und der Gate-Anschluss (11) den Steuereingang (11) des ersten Schaltelements (5) bildet.

7. Aktive Schutzschaltung (1, 77, 79) für einen Elektrodengürtel gemäß Anspruch 2 in Verbindung mit Anspruch 5 oder 6, wobei der Ausgang (9) des ersten Schaltelements (5) mit dem Steuereingang (11) des ersten Schaltelements (5) über das Sperrelement (19) verbunden ist, wobei das Sperrelement (19) so ausgebildet ist, dass die an dem Ausgang (9) des ersten Schaltelements (5) anliegende Spannung nur dann an den Steuereingang (11) des ersten Schaltelements (5) angelegt wird, wenn die am Ausgang (9) des ersten Schaltelements (5) anliegende Spannung in dem Abschaltbereich ist,
wobei das Sperrelement (19) von zwei in Reihe geschalteten Z-Dioden (21, 23) gebildet wird, wobei jede Z-Diode (21, 23) eine Durchlass- und eine Sperrrichtung aufweist und wobei die zwei in Reihe geschalteten Z-Dioden (21, 23) so verschaltet sind, dass die Durchlassrichtung der einen Z-Diode (21) der Sperrrichtung der anderen Z-Diode (23) entspricht.

8. Aktive Schutzschaltung (1) für einen Elektrodengürtel gemäß Anspruch 3 in Verbindung mit Anspruch 7, wobei die aktive Schutzschaltung (1) parallel zu der Schaltelementanordnung (3) die Bypassschaltung (25) aufweist, die den Elektrodeneingang (13) der Schaltelementanordnung (3) mit dem Ausgang (9) des ersten Schaltelements (5) verbindet, wobei die Schaltelementanordnung (3) so ausgebildet ist, das an dem Ausgang (9) des ersten Schaltelements (5) eine Spannung im Abschaltbereich anliegt, wenn das erste Schaltelement (5) keine leitende Verbindung zwischen dem Elektrodeneingang (13) der Schaltelementanordnung (3) und dem Ausgang (9) des ersten Schaltelements (5) ausbildet, und die am Elektrodeneingang (13) der Schaltelementanordnung (3) anliegende Spannung im Hochvoltbereich ist,
wobei die Bypassschaltung (25) zumindest einen hochohmigen Widerstand (27) aufweist.

9. Aktive Schutzschaltung (1, 89, 91) für einen Elektrodengürtel gemäß Anspruch 4 in Verbindung mit einem der Ansprüche 5 bis 7 mit der Speicherschaltung (35), die so ausgebildet ist, dass an dem Steuereingang (11) des ersten Schaltelements (5) dauerhaft eine Spannung angelegt wird, die im zweiten Spannungsbereich ist, wenn an dem Elektrodeneingang (13) einmal eine Spannung anliegt, die im Abschaltbereich ist,
wobei die Speicherschaltung (35) durch ein Flipflop (35) gebildet wird.

10. Aktive Schutzschaltung (1) für einen Elektrodengürtel gemäß einem der Ansprüche 5 bis 9, wobei die Schaltelementanordnung zumindest zwei Steuereingänge (17, 75) und ein zweites Schaltelement (69) mit einem Eingang (71), einem Ausgang (73) und einem Steuereingang (75) aufweist,
wobei ein erster Steuereingang (17) der zumindest zwei Steuereingänge (17, 75) mit dem Steuereingang (11) des ersten Schaltelements (5) verbunden ist,
wobei der Eingang (71) des zweiten Schaltelements (69) mit dem Elektrodeneingang (13) der Schaltelementanordnung (3) verbunden ist, der Ausgang (73) des zweiten Schaltelements (69) mit dem Ausgang (15) der Schaltelementanordnung (3) verbunden ist und der Steuereingang (75) des zweiten Schaltelements (69) mit einem zweiten Steuereingang (75) der zumindest zwei Steuereingänge (17, 75) der Schaltelementanordnung (3) verbunden ist,
wobei das erste und das zweite Schaltelement (5, 69) in Serie geschaltet sind, wobei der Eingang (7, 71) des einen Schaltelements (5, 69) mit dem Ausgang (9, 73) des anderen Schaltelements (7, 71) verbunden ist,
wobei das zweite Schaltelement (69) so ausgebildet ist, dass das zweite Schaltelement (69) eine leitende Verbindung zwischen dem Eingang (71) des zweiten Schaltelements (69) und dem Ausgang (73) des zweiten Schaltelements (69) ausbildet, wenn eine an dem Steuereingang (75) des zweiten Schaltelements (69) anliegende Steuerspannung in einem dritten Spannungsbereich ist, und das zweite Schaltelement (69) keine leitende Verbindung zwischen dem Eingang (71) des zweiten Schaltelements (69) und dem Ausgang (73) des zweiten Schaltelements (69) ausbildet, wenn die an dem Steuereingang (75) des zweiten Schaltelements (69) anliegende Steuerspannung in einem vierten Spannungsbereich ist,
wobei die Schaltelementanordnung (3) so ausgebildet ist, dass die an dem Steuereingang (75) des zweiten Schaltelements (69) anliegende Spannung in den vierten Spannungsbereich wechselt, wenn an dem Elektrodeneingang (13) eine Spannung anliegt, die einen vorbestimmten zweiten Schwellwert unterschreitet.

11. Aktive Schutzschaltung (1) für einen Elektrodengürtel gemäß Anspruch 10, wobei das zweite Schaltelement (69) ein p-Kanal MOSFET (69) mit einem Drain-Anschluss (71), einem Source-Anschluss (73) und einem Gate-Anschluss (75) ist, wobei der Drain-Anschluss (71) den Eingang (71) des zweiten Schaltelements (69) bildet, der Source-Anschluss (73) den Ausgang (73) des zweiten Schaltelements (69) bildet und der Gate-Anschluss (75) den Steuereingang (75) des zweiten Schaltelements (69) bildet.

12. Aktive Schutzschaltung (1, 77, 79, 89, 91) für einen Elektrodengürtel gemäß einem der vorhergehenden Ansprüche, wobei der Abschaltbereich durch eine untere Spannungsgrenze zwischen 40 V und 10 V, vorzugsweise 20 V, definiert ist und sich von dieser unteren Spannungsgrenze als gegen unendlich offener Bereich darstellt.

13. Aktive Schutzschaltung (1, 77, 79, 89, 91) für einen Elektrodengürtel gemäß einem der vorhergehenden Ansprüche, wobei der Abschaltbereich zwei Abschnitte aufweist, wobei ein erster Abschnitt sämtliche Spannungen kleiner zwischen -10 V, vorzugsweise kleiner - 20 V, umfasst, und ein zweiter Abschnitt sämtliche Spannungen größer 10 V, vorzugsweise größer 20 V, umfasst.

## Claims

1. Electrode belt (101) for an electric impedance tomograph, having:
a multiplicity of electrodes (103) that are intended to be arranged on the skin of a patient,
a multiplicity of measuring amplifiers (107), and
a multiplicity of active protective circuits (1, 77, 79, 89, 91) for each of the multiplicity of measuring amplifiers,
wherein each of the multiplicity of electrodes is assigned at least one of the multiplicity of active protective circuits and each of the multiplicity of active protective circuits is assigned to only one of the multiplicity of electrodes,
wherein each of the multiplicity of electrodes (103) is connected to one of the multiplicity of measuring amplifiers by at least one of the multiplicity of active protective circuits,
wherein each of the multiplicity of active protective circuits has a switching element arrangement (3),
wherein the switching element arrangement (3) has an electrode input (13) and an output (15) and at least one control input (17, 75) for a control voltage,
wherein the electrode input (13) of the switching element arrangement (3) is connected to one of the multiplicity of electrodes (103) of the electrode belt (101),
wherein the output (15) of the switching element arrangement (3) is connected to one of the multiplicity of measuring amplifiers (107),
wherein the switching element arrangement (3) is designed such that the switching element arrangement (3) forms a conductive connection between the electrode input (13) of the switching element arrangement (3) and the output (15) of the switching element arrangement (3) when a control voltage present at the at least one control input (17, 75) is in a first voltage range, and the switching element arrangement (3) does not form a conductive connection between the electrode input (13) of the switching element arrangement (3) and the output (15) of the switching element arrangement (3) when the control voltage present at the at least one control input (17, 75) is in a second voltage range, and
wherein each of the multiplicity of active protective circuits (1, 77, 79, 89, 91) is designed such that the voltage present at the at least one control input (17, 75) is in the second voltage range when a voltage that is in a switch-off range is present at the electrode input (13).

2. Active protective circuit (1, 77, 79) for an electrode belt according to Claim 1, wherein the output (15) of the switching element arrangement (3) is connected to the at least one control input (17, 75) of the switching element arrangement by a blocking element (19), wherein the blocking element (19) is designed such that the voltage present at the output (15) of the switching element arrangement (3) is applied to the control input (17, 75) only when the voltage present at the output (15) of the switching arrangement (3) is in the switch-off range.

3. Active protective circuit (1) for an electrode belt according to Claim 2, wherein the active protective circuit (1) has a bypass circuit (25) in parallel with the switching element arrangement (3), which bypass circuit connects the input (13) of the switching element arrangement (3) to the output (15) of the switching element arrangement (3), wherein the bypass circuit (25) is designed such that a voltage in the switch-off range is present at the output (15) of the switching element arrangement (3) when the switching element arrangement (3) does not form a conductive connection between the electrode input (13) of the switching element arrangement (3) and the output (15) of the switching element arrangement (3), and the voltage present at the electrode input (13) of the switching element arrangement (3) is in a high-voltage range, wherein the high-voltage range is a sub-range of the switch-off range.

4. Active protective circuit (1, 89, 91) for an electrode belt according to one of the preceding claims, having a storage circuit (35) that is designed such that a voltage that is in the second voltage range is continuously applied to the at least one control input (17, 75) once a voltage that is in the switch-off range is present at the electrode input (13).

5. Active protective circuit (1, 77, 79, 89, 91) for an electrode belt according to one of the preceding claims, wherein the switching element arrangement (3) has a first switching element (5) having an input (7), an output (9) and a control input (11),
wherein the input (7) of the first switching element (5) is connected to the electrode input (13) of the switching element arrangement (3), the output (9) of the first switching element (5) is connected to the output (15) of the switching element arrangement (3), and the control input (11) of the first switching element (5) is connected to the at least one control input (17, 75) of the switching element arrangement (3),
wherein the first switching element (5) is designed such that the first switching element (5) forms a conductive connection between the input (7) of the first switching element (5) and the output (9) of the first switching element (5) when a control voltage present at the control input (11) of the first switching element (5) is in the first voltage range, and the first switching element (5) does not form a conductive connection between the input (7) of the first switching element (5) and the output (9) of the first switching element (5) when the control voltage present at the control input (11) of the first switching element (5) is in the second voltage range,
wherein the active protective circuit (1, 77, 79, 89, 91) is designed such that the voltage present at the at least one control input (17, 75) changes to the second voltage range when a voltage that exceeds a predetermined first threshold is present at the electrode input (13).

6. Active protective circuit (1, 77, 79, 89, 91) for an electrode belt according to Claim 5, wherein the first switching element (5) is a normally blocking n-channel MOSFET having a drain terminal (7), a source terminal (9) and a gate terminal (11), wherein the drain terminal (7) forms the input (7) of the first switching element (5), the source terminal (9) forms the output (9) of the first switching element (5) and the gate terminal (11) forms the control input (11) of the first switching element (5).

7. Active protective circuit (1, 77, 79) for an electrode belt according to Claim 2 in connection with Claim 5 or 6, wherein the output (9) of the first switching element (5) is connected to the control input (11) of the first switching element (5) by the blocking element (19), wherein the blocking element (19) is designed such that the voltage present at the output (9) of the first switching element (5) is applied to the control input (11) of the first switching element (5) only when the voltage present at the output (9) of the first switching element (5) is in the switch-off range,
wherein the blocking element (19) is formed by two series-connected Zener diodes (21, 23), wherein each Zener diode (21, 23) has a conduction direction and a blocking direction, and wherein the two series-connected Zener diodes (21, 23) are interconnected such that the conduction direction of one Zener diode (21) corresponds to the blocking direction of the other Zener diode (23).

8. Active protective circuit (1) for an electrode belt according to Claim 3 in connection with Claim 7, wherein the active protective circuit (1) has the bypass circuit (25) in parallel with the switching element arrangement (3), which bypass circuit connects the electrode input (13) of the switching element arrangement (3) to the output (9) of the first switching element (5), wherein the switching element arrangement (3) is designed such that a voltage in the switch-off range is present at the output (9) of the first switching element (5) when the first switching element (5) does not form a conductive connection between the electrode input (13) of the switching element arrangement (3) and the output (9) of the first switching element (5), and the voltage present at the electrode input (13) of the switching element arrangement (3) is in the high-voltage range,
wherein the bypass circuit (25) has at least one high-resistance resistor (27).

9. Active protective circuit (1, 89, 91) for an electrode belt according to Claim 4 in connection with one of Claims 5 to 7, having the storage circuit (35) that is designed such that a voltage that is in the second voltage range is continuously applied to the control input (11) of the first switching element (5) once a voltage that is in the switch-off range is present at the electrode input (13), wherein the storage circuit (35) is formed by a flip-flop (35).

10. Active protective circuit (1) for an electrode belt according to one of Claims 5 to 9, wherein the switching element arrangement has at least two control inputs (17, 75) and a second switching element (69) having an input (71), an output (73) and a control input (75),
wherein a first control input (17) of the at least two control inputs (17, 75) is connected to the control input (11) of the first switching element (5),
wherein the input (71) of the second switching element (69) is connected to the electrode input (13) of the switching element arrangement (3), the output (73) of the second switching element (69) is connected to the output (15) of the switching element arrangement (3) and the control input (75) of the second switching element (69) is connected to a second control input (75) of the at least two control inputs (17, 75) of the switching element arrangement (3),
wherein the first and the second switching element (5, 69) are connected in series, wherein the input (7, 71) of one switching element (5, 69) is connected to the output (9, 73) of the other switching element (7, 71),
wherein the second switching element (69) is designed such that the second switching element (69) forms a conductive connection between the input (71) of the second switching element (69) and the output (73) of the second switching element (69) when a control voltage present at the control input (75) of the second switching element (69) is in a third voltage range, and the second switching element (69) does not form a conductive connection between the input (71) of the second switching element (69) and the output (73) of the second switching element (69) when the control voltage present at the control input (75) of the second switching element (69) is in a fourth voltage range,
wherein the switching element arrangement (3) is designed such that the voltage present at the control input (75) of the second switching element (69) changes to the fourth voltage range when a voltage that falls below a predetermined second threshold is present at the electrode input (13).

11. Active protective circuit (1) for an electrode belt according to Claim 10, wherein the second switching element (69) is a p-channel MOSFET (69) having a drain terminal (71), a source terminal (73) and a gate terminal (75), wherein the drain terminal (71) forms the input (71) of the second switching element (69), the source terminal (73) forms the output (73) of the second switching element (69) and the gate terminal (75) forms the control input (75) of the second switching element (69).

12. Active protective circuit (1, 77, 79, 89, 91) for an electrode belt according to one of the preceding claims, wherein the switch-off range is defined by a lower voltage limit between 40 V and 10 V, preferably 20 V, and, from this lower voltage limit, takes the form of a open range tending to infinity.

13. Active protective circuit (1, 77, 79, 89, 91) for an electrode belt according to one of the preceding claims, wherein the switch-off range has two sections, wherein a first section comprises all voltages of less than between -10 V, preferably less than -20 V, and a second section comprises all voltages greater than 10 V, preferably greater than 20 V.

## Revendications

1. Ceinture à électrodes (101) pour un tomographe à impédance électrique comprenant :
une pluralité d'électrodes (103) qui sont prévues pour être disposées sur la peau d'un patient,
une pluralité d'amplificateurs de mesure (107) et
une pluralité de circuits actifs de protection (1, 77, 79, 89, 91) pour chacun de la pluralité d'amplificateurs de mesure,
dans laquelle chacune de la pluralité d'électrodes est associée à au moins l'un de la pluralité de circuits actifs de protection et chacun de la pluralité de circuits actifs de protection est associé à une seule électrode de la pluralité d'électrodes,
dans laquelle chacune de la pluralité d'électrodes (103) est reliée par l'intermédiaire d'au moins l'un de la pluralité de circuits actifs de protection à l'un de la pluralité d'amplificateurs de mesure,
dans laquelle chacun de la pluralité de circuits actifs de protection présente un agencement d'éléments de commutation (3),
dans laquelle l'agencement d'éléments de commutation (3) présente une entrée d'électrode (13) et une sortie (15) et au moins une entrée de commande (17, 75) pour une tension de commande,
dans laquelle l'entrée d'électrode (13) de l'agencement d'éléments de commutation (3) est reliée à l'une de la pluralité électrodes (103) de la ceinture à électrodes (101),
dans laquelle la sortie (15) de l'agencement d'éléments de commutation (3) est reliée à l'un de la pluralité d'amplificateurs de mesure (107),
dans laquelle l'agencement d'éléments de commutation (3) est conçu de telle sorte que l'agencement d'éléments de commutation (3) forme une liaison conductrice entre l'entrée d'électrode (13) de l'agencement d'éléments de commutation (3) et la sortie (15) de l'agencement d'éléments de commutation (3) lorsqu'une tension de commande appliquée à ladite au moins une entrée de commande (17, 75) se situe dans une première plage de tension, et l'agencement d'éléments de commutation (3) ne forme pas de liaison conductrice entre l'entrée d'électrode (13) de l'agencement d'éléments de commutation (3) et la sortie (15) de l'agencement d'éléments de commutation (3) lorsque la tension de commande appliquée à ladite au moins une entrée de commande (17, 75) se situe dans une deuxième plage de tension, et
dans laquelle chacun de la pluralité de circuits actifs de protection (1, 77, 79, 89, 91) est configuré de telle sorte que la tension appliquée à ladite au moins une entrée de commande (17, 75) se situe dans la deuxième plage de tension lorsqu'une tension appliquée à l'entrée d'électrode (13) se situe dans une plage de coupure.

2. Circuit actif de protection (1, 77, 79) pour une ceinture à électrodes selon la revendication 1, dans lequel la sortie (15) de l'agencement d'éléments de commutation (3) est reliée à ladite au moins une entrée de commande (17, 75) de l'agencement d'éléments de commutation par l'intermédiaire d'un élément de blocage (19), l'élément de blocage (19) étant réalisé de telle sorte que la tension appliquée à la sortie (15) de l'agencement d'éléments de commutation (3) n'est appliquée à l'entrée de commande (17, 75) que lorsque la tension appliquée à la sortie (15) de l'agencement d'éléments de commutation (3) se situe dans la plage de coupure.

3. Circuit actif de protection (1) pour une ceinture à électrodes selon la revendication 2, lequel circuit actif de protection (1) présente, en parallèle à l'agencement d'éléments de commutation (3), un circuit de dérivation (25) qui relie l'entrée (13) de l'agencement d'éléments de commutation (3) à la sortie (15) de l'agencement d'éléments de commutation (3), le circuit de dérivation (25) étant réalisé de telle sorte qu'une tension dans la plage de coupure est appliquée à la sortie (15) de l'agencement d'éléments de commutation (3) lorsque l'agencement d'éléments de commutation (3) ne forme pas de liaison conductrice entre l'entrée d'électrode (13) de l'agencement d'éléments de commutation (3) et la sortie (15) de l'agencement d'éléments de commutation (3), et que la tension appliquée à l'entrée d'électrode (13) de l'agencement d'éléments de commutation (3) se situe dans une plage de haute tension, la plage de haute tension constituant une partie de la plage de coupure.

4. Circuit actif de protection (1, 89, 91) pour une ceinture à électrodes selon l'une des revendications précédentes, comprenant un circuit de mémoire (35) qui est réalisé de telle sorte qu'une tension qui se situe dans la deuxième plage de tension est appliquée de manière permanente à ladite au moins une entrée de commande (17, 75) lorsqu'une tension qui se situe dans la plage de coupure est appliquée une fois à l'entrée d'électrode (13).

5. Circuit actif de protection (1, 77, 79, 89, 91) pour une ceinture à électrodes selon l'une des revendications précédentes, dans lequel l'agencement d'éléments de commutation (3) présente un premier élément de commutation (5) avec une entrée (7), une sortie (9) et une entrée de commande (11),
dans lequel l'entrée (7) du premier élément de commutation (5) est reliée à l'entrée d'électrode (13) de l'agencement d'éléments de commutation (3), la sortie (9) du premier élément de commutation (5) est reliée à la sortie (15) de l'agencement d'éléments de commutation (3) et l'entrée de commande (11) du premier élément de commutation (5) est reliée à ladite au moins une entrée de commande (17, 75) de l'agencement d'éléments de commutation (3),
dans lequel le premier élément de commutation (5) est conçu de telle sorte que le premier élément de commutation (5) forme une liaison conductrice entre l'entrée (7) du premier élément de commutation (5) et la sortie (9) du premier élément de commutation (5) lorsqu'une tension de commande appliquée à l'entrée de commande (11) du premier élément de commutation (5) se situe dans la première plage de tension et que le premier élément de commutation (5) ne forme pas de liaison conductrice entre l'entrée (7) du premier élément de commutation (5) et la sortie (9) du premier élément de commutation (5) lorsque la tension de commande appliquée à l'entrée de commande (11) du premier élément de commutation (5) se situe dans la deuxième plage de tension,
dans lequel le circuit actif de protection (1, 77, 79, 89, 91) est réalisé de telle sorte que la tension appliquée à ladite au moins une entrée de commande (17, 75) passe dans la deuxième plage de tension lorsqu'une tension appliquée à l'entrée d'électrode (13) dépasse une première valeur seuil prédéterminée.

6. Circuit actif de protection (1, 77, 79, 89, 91) pour une ceinture à électrodes selon la revendication 5, dans lequel le premier élément de commutation (5) est un MOSFET à canal n normalement bloquant avec une borne de drain (7), une borne de source (9) et une borne de grille (11), la borne de drain (7) formant l'entrée (7) du premier élément de commutation (5), la borne de source (9) formant la sortie (9) du premier élément de commutation (5) et la borne de grille (11) formant l'entrée de commande (11) du premier élément de commutation (5).

7. Circuit actif de protection (1, 77, 79) pour une ceinture à électrodes selon la revendication 2 en liaison avec la revendication 5 ou 6, dans lequel la sortie (9) du premier élément de commutation (5) est reliée à l'entrée de commande (11) du premier élément de commutation (5) par l'intermédiaire de l'élément de blocage (19), l'élément de blocage (19) étant réalisé de telle sorte que la tension appliquée à la sortie (9) du premier élément de commutation (5) n'est appliquée à l'entrée de commande (11) du premier élément de commutation (5) que lorsque la tension appliquée à la sortie (9) du premier élément de commutation (5) se situe dans la plage de coupure,
dans lequel l'élément de blocage (19) est formé par deux diodes Z (21, 23) montées en série, chaque diode Z (21, 23) ayant un sens de passage et un sens de blocage et les deux diodes Z (21, 23) montées en série étant branchées de telle sorte que le sens de passage d'une diode Z (21) correspond au sens de blocage de la deuxième diode Z (23).

8. Circuit actif de protection (1) pour une ceinture à électrodes selon la revendication 3 en liaison avec la revendication 7, dans lequel le circuit actif de protection (1) comprend, en parallèle à l'agencement d'éléments de commutation (3), le circuit de dérivation (25) qui relie l'entrée d'électrode (13) de l'agencement d'éléments de commutation (3) et la sortie (9) du premier élément (5), l'agencement d'éléments de commutation (3) étant réalisé de telle sorte qu'une tension dans la plage de coupure est appliquée à la sortie (9) du premier élément de commutation (5) lorsque le premier élément de commutation (5) ne forme pas de liaison conductrice entre l'entrée d'électrode (13) de l'agencement d'éléments de commutation (3) et la sortie (9) du premier élément de commutation (5), et que la tension appliquée à l'entrée d'électrode (13) de l'agencement d'éléments de commutation (3) se situe dans la plage de haute tension,
dans lequel le circuit de dérivation (25) présente au moins une résistance de valeur ohmique élevée (27).

9. Circuit actif de protection (1, 89, 91) pour une ceinture à électrodes selon la revendication 4 en liaison avec l'une des revendications 5 à 7, avec le circuit de mémoire (35) qui est réalisé de telle sorte qu'une tension qui se situe dans la deuxième plage de tension est appliquée de manière permanente à l'entrée de commande (11) du premier élément de commutation (5) lorsqu'une tension qui se situe dans la plage de coupure est appliquée une fois à l'entrée d'électrode (13),
dans lequel le circuit de mémoire (35) est formé par un flip-flop (35).

10. Circuit actif de protection (1) pour une ceinture à électrodes selon l'une des revendications 5 à 9, dans lequel l'agencement d'éléments de commutation présente au moins deux entrées de commande (17, 75) et un deuxième élément de commutation (69) avec une entrée (71), une sortie (73) et une entrée de commande (75),
dans lequel une première entrée de commande (17) desdites au moins deux entrées de commande (17, 75) est reliée à l'entrée de commande (11) du premier élément de commutation (5),
dans lequel l'entrée (71) du deuxième élément de commutation (69) est reliée à l'entrée d'électrode (13) de l'agencement d'éléments de commutation (3), la sortie (73) du deuxième élément de commutation (69) est reliée à la sortie (15) de l'agencement d'éléments de commutation (3) et l'entrée de commande (75) du deuxième élément de commutation (69) est reliée à une deuxième entrée de commande (75) desdites au moins deux entrées de commande (17, 75) de l'agencement d'éléments de commutation (3),
dans lequel les premier et deuxième éléments de commutation (5, 69) sont montés en série, l'entrée (7, 71) d'un élément de commutation (5, 69) étant reliée à la sortie (9, 73) de l'autre élément de commutation (7, 71),
dans lequel le deuxième élément de commutation (69) est réalisé de telle sorte que le deuxième élément de commutation (69) forme une liaison conductrice entre l'entrée (71) du deuxième élément de commutation (69) et la sortie (73) du deuxième élément de commutation (69) lorsqu'une tension de commande appliquée à l'entrée de commande (75) du deuxième élément de commutation (69) se situe dans une troisième plage de tension et que le deuxième élément de commutation (69) ne forme pas de liaison conductrice entre l'entrée (71) du deuxième élément de commutation (69) et la sortie (73) du deuxième élément de commutation (69) lorsque la tension de commande appliquée à l'entrée de commande (75) du deuxième élément de commutation (69) se situe dans une quatrième plage de tension,
dans lequel l'agencement d'éléments de commutation (3) est réalisé de telle sorte que la tension appliquée à l'entrée de commande (75) du deuxième élément de commutation (69) passe dans la quatrième plage de tension lorsqu'une tension appliquée à l'entrée d'électrode (13) devient inférieure à une deuxième valeur seuil prédéterminée.

11. Circuit actif de protection (1) pour une ceinture à électrodes selon la revendication 10, dans lequel le deuxième élément de commutation (69) est un MOSFET à canal p (69) avec une borne de drain (71), une borne de source (73) et une borne de grille (75), la borne de drain (71) formant l'entrée (71) du deuxième élément de commutation (69), la borne de source (73) formant la sortie (73) du deuxième élément de commutation (69) et la borne de grille (75) formant l'entrée de commande (75) du deuxième élément de commutation (69).

12. Circuit actif de protection (1, 77, 79, 89, 91) pour une ceinture à électrodes selon l'une des revendications précédentes, dans lequel la plage de coupure est définie par une limite de tension inférieure comprise entre 40 V et 10 V, de préférence de 20 V, et se présente sous la forme d'une plage ouverte vers l'infini à partir de cette limite de tension inférieure.

13. Circuit actif de protection (1, 77, 79, 89, 91) pour une ceinture à électrodes selon l'une des revendications précédentes, dans lequel la plage de coupure présente deux sections, une première section comprenant toutes les tensions inférieures à - 10 V, de préférence inférieures à - 20 V, et une deuxième section comprenant toutes les tensions supérieures à 10 V, de préférence supérieures à 20 V.
